(19) Europäisches Patentamt
European Patent Office
Office européen des brevets

(11) EP 4 755 401 A1

(12) **EUROPEAN PATENT APPLICATION**
published in accordance with Art. 153(4) EPC

(43) Date of publication:
**10.06.2026 Bulletin 2026/24**

(21) Application number: **24848173.1**

(22) Date of filing: **26.07.2024**

(51) International Patent Classification (IPC):
*A61K 47/68* (2017.01) *A61K 31/58* (2006.01)
*C07K 16/22* (2006.01) *A61P 37/00* (2006.01)

(52) Cooperative Patent Classification (CPC):
**A61K 31/58; A61K 47/68; A61P 37/00; C07K 16/22**

(86) International application number:
**PCT/CN2024/107802**

(87) International publication number:
**WO 2025/026216 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.07.2023 CN 202310938284**

(71) Applicant: **Jiangsu Hengrui Pharmaceuticals Co., Ltd.**
**Lianyungang, Jiangsu 222047 (CN)**

(72) Inventors:
- **ZHOU, Xiaodan**
  **Lianyungang, Jiangsu 222047 (CN)**
- **LI, Jinyu**
  **Lianyungang, Jiangsu 222047 (CN)**
- **YE, Xiu**
  **Lianyungang, Jiangsu 222047 (CN)**
- **SUN, Qiong**
  **Lianyungang, Jiangsu 222047 (CN)**
- **LU, Yun**
  **Lianyungang, Jiangsu 222047 (CN)**
- **YANG, Jian**
  **Lianyungang, Jiangsu 222047 (CN)**

(74) Representative: **dompatent**
**Partnerschaft von**
**Patentanwälten und Rechtsanwälten mbB**
**Deichmannhaus am Dom**
**Bahnhofsvorplatz 1**
**50667 Köln (DE)**

Remarks:
The complete document including Reference Table(s) and the Sequence Listing(s) can be downloaded from the EPO website

# (54) PHARMACEUTICAL COMPOSITION CONTAINING ANTIBODY-DRUG CONJUGATE OF GLUCOCORTICOID

(57) A pharmaceutical composition containing an antibody-drug conjugate of glucocorticoid. Specifically, the present invention relates to a pharmaceutical composition, comprising an antibody-drug conjugate and a counterion. The pharmaceutical composition has good aerodynamic characteristics and is suitable for inhalation administration.

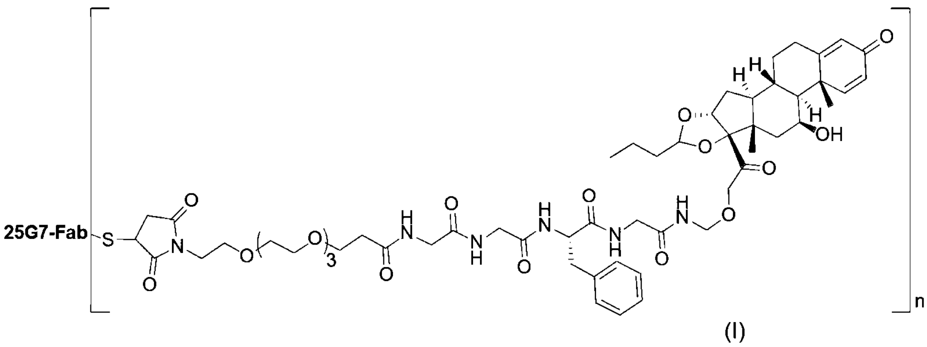

(I)



Processed by Luminess, 75001 PARIS (FR)

## Description

The present application claims the priority of Chinese patent application 2023109382848 filed on July 28, 2023. This Chinese patent application is incorporated herein by reference in its entirety.

## Technical Field

The present disclosure belongs to the field of pharmaceutical preparations, and specifically relates to a pharmaceutical composition comprising an antibody-drug conjugate of a glucocorticoid.

## Background Art

Interleukin-4 (IL-4) consists of 153 amino acids and has a molecular weight of about 17 kDa. Initially, IL-4 was found for its ability to stimulate B cell proliferation, and was therefore named B cell-stimulating factor-1 (BSF-1). IL-4, like IL-13, belongs to the type I cytokine family and has a quaternary structure composed of a hydrophobic bundle core with 4 α-helices. IL-4 is secreted by TH2 cells, participates in TH2-mediated immune response, and exhibits broad biological activities, including stimulating the proliferation of T cells, mast cells, granulocytes, megakaryocytes, and erythrocytes. In addition, IL-4 can also stimulate B cells to express major histocompatibility complex class 2 molecules. IL-13 shares approximately 30% amino acid sequence homology with IL-4 and exhibits multiple similar functions. Both IL-4 and IL-13 can promote B cell proliferation, and together with CD40/CD40L costimulation, induce the class switching from IgM to IgE. IL-4 promotes mast cell aggregation, upregulates the expression of the high-affinity IgE receptor on mast cells and the low-affinity IgE receptor CD23 (FcεRII) on B cells, upregulates the expression of vascular cell adhesion molecule (VCAM-1) on vascular endothelial cells, and promotes the transfer of eosinophils, T lymphocytes, monocytes, and basophils. Unlike IL-13, IL-4 can promote the differentiation of naive T cells into TH2.

IL-4 needs to bind to membrane receptors to exert its biological functions. The human interleukin receptor (IL-4R) is a heterodimer formed by two polypeptide chains. One of these chains is the α chain, which has high affinity for IL-4. Since the IL-4Rα chain within the IL-4R complex plays a dominant role in binding to IL-4, IL-4Rα is often used as a substitute for IL-4R in numerous scientific studies and reports. IL-4R is expressed on various cells, including human B cells, mast cells, eosinophils, basophils, macrophages/monocytes, DC cells, fibrocytes, and airway epithelial and smooth muscle cells. IL-4Rα can form two types of receptor complexes with other subunits, and the type I receptor composed of IL-4Rα and γc is mainly expressed in hematopoietic stem cells. In non-hematopoietic stem cells, IL-4 acts primarily through the type II receptor composed of IL-4Rα and IL-13Rα1. The type II receptor is a co-receptor for IL-4 and IL-13, and IL-13 functions by binding to IL-13Rα1. Both type I and type II receptors transduce signals through the Jak/STAT pathway, and IL-4Rα, γc and IL-13Rα1 bind to Jak1, Jak3 and Tyk2, respectively, to activate downstream pathways. IL-4 and IL-13 can also transduce signals through the insulin receptor substrate family (IRS), ultimately activating PI3-K and NF-κB in the nucleus. Blocking IL-4R can inhibit the biological functions of both IL-4 and IL-13.

Several studies have shown that IL-4 and IL-13 are associated with TH2 immune response-related diseases. Atopic dermatitis (AD), also known as atopic skin inflammation or hereditary allergic dermatitis, is a common dermatological disease, most commonly found in children and adolescents. It is often comorbid with some hereditary allergic diseases such as allergic rhinitis and asthma. Studies have found that TH2 factors IL-4, IL-5, IL-10, and IL-13 in AD patients have elevated levels of IgE. In addition, it has also been found that the TH2 factors are associated with AD disease progression, and mice overexpressing TH2 factors such as IL-4 and IL-13 exhibit skin protection deficits and AD-like conditions [14][15]. Elevated levels of IL-4 and IL-13 in AD patients impede epidermal differentiation and production of antimicrobial peptides. IL-4 deficient mice have reduced development of cutaneous allergic inflammation. These studies have shown that blocking IL-4R may be effective in treating AD. Monoclonal antibodies against IL-4R have been marketed overseas and exhibit favorable therapeutic effects for AD.

IL-13 and IL-4 also play important roles in asthma. Asthma is a common pulmonary inflammatory disease characterized by airway hyperresponsiveness (AHR), mucus hypersecretion, fibrosis and elevated IgE levels. Non-specific stimuli such as cold air often lead to increased airway hyperresponsiveness, and AHR and mucus hypersecretion lead to airway obstruction, which is a major cause of death in asthma. TH2 factors play an important role in the disease progression of asthma, and IL-4 and IL-13 are overexpressed in bronchoalveolar lavage fluid of asthma patients. Despite some functional similarities between IL-13 and IL-4, some studies have shown that IL-13 plays a more important role in the disease progression of asthma than other Th2 cytokines. IL-13 can promote goblet cell differentiation and fibrosis. Injection of recombinant IL-13 to the airways of mice not stimulated with allergens will cause airway inflammation, mucus hypersecretion and airway hyperresponsiveness. Injection of soluble IL13Rα2 can prevent the development of AHR, mucus hypersecretion and pulmonary inflammation in mice. In asthma models, injection of IL-4Rα antibodies can reduce AHR and eosinophils in bronchoalveolar lavage fluid. Studies have shown that blocking IL-4Rα may be effective in treating asthma.

Monoclonal antibodies against IL-4R are currently being developed by numerous pharmaceutical companies in various countries, with related patent applications such as WO 2010053751, WO 2001092340, WO 2008054606, WO 2014031610 and WO 2020038454.

Glucocorticoids are also relatively effective drugs for treating allergic diseases, inflammation, and the like. As representative glucocorticoids, cortisol, corticosterone, and other glucocorticoids produced in organisms, as well as synthetic glucocorticoids such as dexamethasone, prednisone, prednisolone, and budesonide, are known. These glucocorticoids are collectively referred to as steroids due to their steroid structure, and are used in the treatment of various diseases. However, these steroids sometimes exhibit steroid peptic ulcers, steroid purple spots, steroid pancreatitis, steroid diabetes, steroid cataracts, steroid glaucoma and other side effects due to their use.

Antibody-drug conjugates (ADCs) are monoclonal antibodies or antibody fragments that are linked to a biologically active drug via a stable chemical linker compound. Most ADCs in preclinical and clinical development are used for oncology indications, with cytotoxic payloads targeting cancer cells that express antigens. However, regulating the activity of pathogenic cells through ADC-mediated delivery of bioactive small molecules is also attractive for non-oncology indications, thus leading to the widespread application of this technology.

Some drug conjugates of glucocorticoids have been disclosed in the prior art, such as WO 2017210471, WO 2019106609, and WO 2019136487.

**Summary of the Invention**

The present disclosure provides a pharmaceutical composition, comprising an antibody-drug conjugate and a counterion, wherein the antibody-drug conjugate has the structure as shown in formula I:

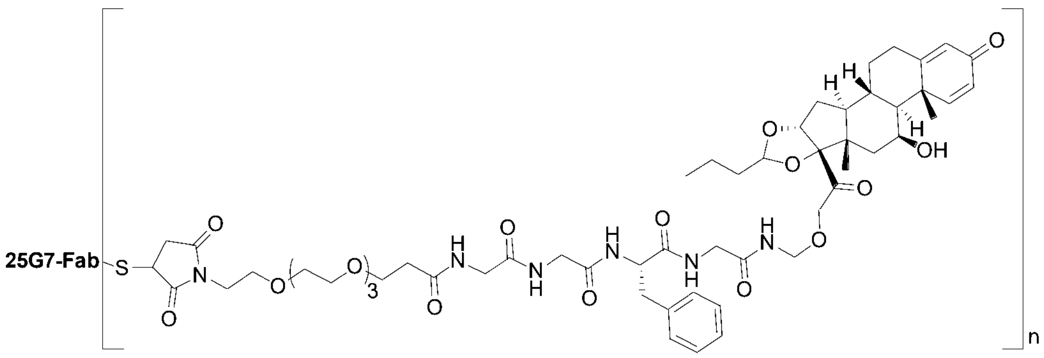

I ;

wherein:

25G7-Fab comprises a heavy chain variable region and a light chain variable region, the heavy chain variable region comprising: HCDR1 as set forth in SEQ ID NO: 1 or SEQ ID NO: 12, HCDR2 as set forth in SEQ ID NO: 2, and HCDR3 as set forth in SEQ ID NO: 3, the light chain variable region comprising: LCDR1 as set forth in SEQ ID NO: 4, LCDR2 as set forth in SEQ ID NO: 5, and LCDR3 as set forth in SEQ ID NO: 6; and
n is 1 to 10.

In some embodiments, the range of the average drug load (n) may be the average number of glucocorticoid drugs conjugated to each 25G7-Fab. Non-limiting examples include an average of 1, 2, 3, 4, 5, 6, 7, 8, 9, or 10 glucocorticoid drugs conjugated to each 25G7-Fab, and any range between these point values. Examples can be 2-8, 2-7, 2-6, 2-5, 2-4, 3-4, 3-5, 3.5-4.7, 5-6, 5-7, 5-8 and 6-8. Exemplarily, the average drug load (n) may be an average of 1, 2, 3, 4, 5, 6, 7, 8, 9, or 10. n is a decimal number or an integer.

In some embodiments, the 25G7-Fab has a heavy chain variable region as set forth in SEQ ID NO: 8, and a light chain variable region as set forth in SEQ ID NO: 7.

In some embodiments, the 25G7-Fab has a heavy chain as set forth in SEQ ID NO: 11 and a light chain as set forth in SEQ ID NO: 10.

The CDR sequences of 25G7-Fab are shown in Table 1:

Table 1. CDR sequences of 25G7-Fab

| Name | Sequence | No. |
|---|---|---|
| HCDR1 | DYGMH | SEQ ID NO: 1 |
| HCDR2 | FISSGSSIIYYADIVKG | SEQ ID NO: 2 |
| HCDR3 | GNKRGFFDY | SEQ ID NO: 3 |
| LCDR1 | RASSSVPYMY | SEQ ID NO: 4 |
| LCDR2 | LTSNLAS | SEQ ID NO: 5 |
| LCDR3 | QQWRAYPPMLT | SEQ ID NO: 6 |
| HCDR1 | GFTFSDYGMH | SEQ ID NO: 12 |

For 25G7-Fab, the sequence of the light chain variable region is as set forth in SEQ ID NO: 7 and the sequence of the heavy chain variable region is as set forth in SEQ ID NO: 8.

hu25G7-A LCVR (hu25G7-A light chain variable region)

EIVLTQSPATLSLSPGERATLSCRASSSVPYMYWYQQKPGQAPRLLIY
LTSNLASGIPARFSGSGSGTDFTLTISSLEPEDFAVYYCQQWRAYPPMLTFG
GGTKVEIK
SEQ ID NO: 7

hu25G7-VH (hu25G7 heavy chain variable region)

EVQLVESGGGLVQPGGSLRLSCAASGFTFSDYGMHWVRQAPGKGLE
WVAFISSGSSIIYYADIVKGRSTISRDNAKNTLYLQMNSLRAEDTAVYYCT
RGNKRGFFDYWGQGTLVTVSS
SEQ ID NO: 8

For 25G7-Fab, the sequence of the light chain is as set forth in SEQ ID NO: 10 and the sequence of the heavy chain is as set forth in SEQ ID NO: 11; and for hu25G7, the sequence of the heavy chain is as set forth in SEQ ID NO: 9 and the sequence of the light chain is as set forth in SEQ ID NO: 10.

hu25G7-IgG4 HC

EVQLVESGGGLVQPGGSLRLSCAASGFTFSDYGMHWVRQAPGKGLE
WVAFISSGSSIIYYADIVKGRSTISRDNAKNTLYLQMNSLRAEDTAVYYCT
RGNKRGFFDYWGQGTLVTVSSASTKGPSVFPLAPCSRSTSESTAALGCLVK
DYFPEPVTVSWNSGALTSGVHTFPAVLQSSGLYSLSSVVTVPSSSLGTKTY
TCNVDHKPSNTKVDKRVESKYGPPCPPCPAPEFLGGPSVFLFPPKPKDTLMI
SRTPEVTCVVVDVSQEDPEVQFNWYVDGVEVHNAKTKPREEQFNSTYRV
VSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGS
FFLYSRLTVDKSRWQEGNVFSCSVMHEALHNHYTQKSLSLSLGK

SEQ ID NO: 9

hu25G7-A LC

EIVLTQSPATLSLSPGERATLSCRASSSVPYMYWYQQKPGQAPRLLIY
LTSNLASGIPARFSGSGSGTDFTLTISSLEPEDFAVYYCQQWRAYPPMLTFG
GGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQG
LSSPVTKSFNRGEC

SEQ ID NO: 10

hu25G7-24-IgG4 VH-CH1

EVQLVESGGGLVQPGGSLRLSCAASGFTFSDYGMHWVRQAPGKGLE
WVAFISSGSSIIYYADIVKGRSTISRDNAKNTLYLQMNSLRAEDTAVYYCT
RGNKRGFFDYWGQGTLVTVSSASTKGPSVFPLAPCSRSTSESTAALGCLVK
DYFPEPVTVSWNSGALTSGVHTFPAVLQSSGLYSLSSVVTVPSSSLGTKTY
TCNVDHKPSNTKVDKRV

SEQ ID NO: 11

In some embodiments, the concentration of the antibody-drug conjugate is 0.1 mg/mL-50 mg/mL based on the protein concentration, and non-limiting examples include 0.1 mg/mL, 0.5 mg/mL, 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 6.0 mg/mL, 7.0 mg/mL, 8.0 mg/mL, 9.0 mg/mL, 10.0 mg/mL, 15.0 mg/mL, 20.0 mg/mL, 25.0 mg/mL, 30.0 mg/mL, 35.0 mg/mL, 40.0 mg/mL, 45.0 mg/mL, 50.0 mg/mL, and any range between these point values. In some embodiments, the concentration of the antibody-drug conjugate is 0.5 mg/mL-10 mg/mL. In some embodiments, the concentration of the antibody-drug conjugate is 1 mg/mL-5 mg/mL.

In some embodiments, the counterion includes, but is not limited to, sulfate, phosphate, malate, maleate, oxalate, citrate, succinate, fumarate, itaconate, chloride, bromide, etc. In some embodiments, the counterion is selected from one or more of sulfate, citrate, and chloride. In some embodiments, the counterion is selected from one or more of sodium citrate, sodium sulfate, zinc sulfate, magnesium sulfate, potassium sulfate, calcium sulfate, magnesium chloride, calcium chloride and zinc chloride. In some embodiments, the counterion is sodium sulfate and/or sodium citrate.

In some embodiments, the concentration of the counterion is 0.01 mg/mL-10 mg/mL, and non-limiting examples include 0.01 mg/mL, 0.05 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, 1.6 mg/mL, 1.7 mg/mL, 1.8

mg/mL, 1.9 mg/mL, 2.0 mg/mL, 2.2 mg/mL, 2.4 mg/mL, 2.6 mg/mL, 2.8 mg/mL, 3.0 mg/mL, 4.0 mg/mL, 5.0 mg/mL, 6.0 mg/mL, 7.0 mg/mL, 8.0 mg/mL, 9.0 mg/mL, 10.0 mg/mL, and any range between these point values. In some embodiments, the concentration of the counterion is 0.05 mg/mL-5 mg/mL. In some embodiments, the concentration of the counterion is 0.1 mg/mL-2 mg/mL.

In some embodiments, the pharmaceutical composition further comprises a protective agent. In some embodiments, the protective agent is selected from an amino acid and/or a sugar.

In some embodiments, the amino acids include, but are not limited to, glycine, histidine, arginine, lysine, glutamic acid, alanine, valine, leucine, isoleucine, proline, tryptophan, phenylalanine, methionine, aspartic acid, etc. In some embodiments, the amino acid is glycine or histidine.

In some embodiments, the concentration of the amino acid is 0.01 mg/mL-10 mg/mL, and non-limiting examples include 0.01 mg/mL, 0.05 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, 1.6 mg/mL, 1.7 mg/mL, 1.8 mg/mL, 1.9 mg/mL, 2.0 mg/mL, 2.2 mg/mL, 2.4 mg/mL, 2.6 mg/mL, 2.8 mg/mL, 3.0 mg/mL, 4.0 mg/mL, 5.0 mg/mL, 6.0 mg/mL, 7.0 mg/mL, 8.0 mg/mL, 9.0 mg/mL, 10.0 mg/mL, and any range between these point values. In some embodiments, the concentration of the amino acid is 0.05 mg/mL-5 mg/mL. In some embodiments, the concentration of the amino acid is 0.1 mg/mL-2 mg/mL.

The "sugar" of the present disclosure includes a conventional compound of formula $(CH_2O)_n$ and a derivative thereof, including monosaccharides, disaccharides, trisaccharides, polysaccharides, sugar alcohols, reducing sugars, non-reducing sugars, and the like. In some embodiments, the sugar includes, but is not limited to, sorbitol, mannitol, xylitol, trehalose, lactose, fructose, maltose, sucrose, etc. In some embodiments, the sugar is trehalose.

In some embodiments, the concentration of the sugar is 0.01 mg/mL-10 mg/mL, and non-limiting examples include 0.01 mg/mL, 0.05 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, 1.6 mg/mL, 1.7 mg/mL, 1.8 mg/mL, 1.9 mg/mL, 2.0 mg/mL, 2.2 mg/mL, 2.4 mg/mL, 2.6 mg/mL, 2.8 mg/mL, 3.0 mg/mL, 4.0 mg/mL, 5.0 mg/mL, 6.0 mg/mL, 7.0 mg/mL, 8.0 mg/mL, 9.0 mg/mL, 10.0 mg/mL, and any range between these point values. In some embodiments, the concentration of the sugar is 0.05 mg/mL-5 mg/mL. In some embodiments, the concentration of the sugar is 0.1 mg/mL-2 mg/mL.

In some embodiments, the pharmaceutical composition further comprises an organic solvent, and the organic solvent includes, but is not limited to, methanol, ethanol, 1-propanol, isopropanol, tert-butanol, butanol, acetone, acetonitrile, acetic acid, ethyl acetate, methyl ethyl ketone, methyl tert-butyl ether, dimethyl sulfoxide and the like. In some embodiments, the organic solvent is isopropanol.

In some embodiments, the amount of the organic solvent is 0.1%-50% v/v, and non-limiting examples include 0.1%, 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 11.0%, 12.0%, 13.0%, 14.0%, 15.0%, 20.0%, 25.0%, 30.0%, 35.0%, 40.0%, 45.0%, 50.0%, and any range between these point values. In some embodiments, the amount of the organic solvent is 0.5%-30% v/v. In some embodiments, the amount of the organic solvent is 1%-10% v/v.

In some embodiments, the pH of the pharmaceutical composition is 3.0-8.0, and non-limiting examples include 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, and any range between these point values. In some embodiments, the pH is 4.5-6.0. In some embodiments, the pH is 5.0 or 5.5.

In some embodiments, the pharmaceutical composition comprises:

a) 1 mg/mL-5 mg/mL of the antibody-drug conjugate based on the protein concentration,
b) 0.1 mg/mL-2 mg/mL of the counterion which is sodium citrate or sodium sulfate,
c) 0.1 mg/mL-2 mg/mL of the amino acid which is histidine or glycine,
d) 0.1 mg/mL-2 mg/mL of the sugar which is trehalose, and
e) 1%-10% v/v of the organic solvent which is isopropanol;

and the pharmaceutical composition has a pH of 4.5-5.5.

The present disclosure further provides a lyophilized preparation comprising the antibody-drug conjugate, wherein the preparation, upon reconstitution, can form the pharmaceutical composition as described above.

The present disclosure further provides a method for preparing a lyophilized preparation comprising the antibody-drug conjugate, wherein the method comprises the step of freeze-drying the pharmaceutical composition as described above.

The present disclosure further provides a lyophilized preparation comprising the antibody-drug conjugate, and the lyophilized preparation is obtained by freeze-drying the pharmaceutical composition of the antibody-drug conjugate as described above.

In some embodiments, the lyophilized preparation is in the form of microspheres. In some embodiments, the

microspheres have an aerodynamic particle size of 0.01-10.0 μm. In some embodiments, the microspheres have an aerodynamic particle size of 0.5-5 μm.

The present disclosure further provides a reconstituted solution comprising an antibody-drug conjugate, wherein the reconstituted solution is prepared by reconstituting the lyophilized preparation as described above.

The present disclosure further provides a method for preparing the reconstituted solution described above, wherein the method comprises the step of reconstituting the lyophilized preparation described above, wherein the solution used for the reconstitution is selected from, but not limited to water for injection, physiological saline or glucose solution.

The present disclosure further provides a pharmaceutical composition comprising the lyophilized preparation described previously, and a filler including, but not limited to, trehalose, lactose, mannitol, glucose, sorbitol, dextran, etc. In some embodiments, the filler is trehalose.

The present disclosure further provides an article of manufacture comprising a container, wherein the container contains the pharmaceutical composition, lyophilized preparation, reconstituted solution or dry powder preparation as described above. In some embodiments, the container is a neutral borosilicate glass tubular injection vial.

The present disclosure further provides the use of the pharmaceutical composition, lyophilized preparation, reconstituted solution or article of manufacture described above in the preparation of a medicament for treating or preventing an immune disease or condition.

The present disclosure further provides a method for treating or preventing an immune disease or condition, comprising administering to a subject an effective amount or a prophylactically effective amount of the pharmaceutical composition, lyophilized preparation, reconstituted solution or article of manufacture described above.

In some embodiments, the disease or condition is selected from: asthma, nasal polyps, chronic sinusitis, allergic skin disease, eosinophilic esophagitis, chronic obstructive pulmonary disease, allergic rhinitis, arthritis, inflammatory diseases, allergic reactions, autoimmune lymphoproliferative syndrome, autoimmune hemolytic anemia, Barrett's esophagus, autoimmune uveitis, tuberculosis and kidney disease. In some embodiments, the disease or condition is selected from asthma or allergic skin disease.

## Brief Description of the Drawings

FIG. 1: Dose-response curves of whole cell binding activity between the test proteins and human-derived IL-4Rα expressing cells.

FIG. 2: Dose-response curve of the test proteins for blocking the IL-4/IL-13 signaling pathway.

FIG. 3: Endocytosis rate-time curves of the test proteins in TF-1 cells.

FIG. 4: Time-concentration curves of ADC-2 and free budesonide in various tissues after intratracheal administration.

FIG. 5: In test example 6, counts of leukocytes and classified cell types in bronchoalveolar lavage fluid.

FIG. 6: In test example 7, counts of leukocytes and classified cell types in bronchoalveolar lavage fluid.

FIG. 7: Scanning electron micrograph of dry powder formulation c for inhalation.

FIG. 8: Scanning electron micrograph of dry powder formulation f for inhalation.

## Terms

To facilitate a better understanding of the present disclosure, certain technical and scientific terms are specifically defined below. Unless otherwise explicitly defined herein, all other technical and scientific terms used herein have the meanings commonly understood by those of ordinary skill in the art to which the present disclosure pertains.

The term "antibody-drug conjugate" refers to a ligand linked to a biologically active drug via a stable linking unit. In the present disclosure, the "antibody-drug conjugate (ADC)" refers to a monoclonal antibody or antibody fragment linked to a biologically active glucocorticoid via a stable linking unit. In the conjugate, the antibody or antibody fragment can bind to the glucocorticoid molecule containing a linker via a specific group therein (e.g., an interchain disulfide bond).

The term "counterion" refers to a charged or charge-polarized molecule capable of initiating the formation of microparticles from macromolecules such as proteins, nucleic acids, lipids, or oligosaccharides. Whether a charged molecule is a counterion can be empirically determined based on the following parameters, including but not limited to the type of protein, pH, ionic strength, the type of organic solvent used, and the presence of salts and other components such as active reagents. As provided and described herein, a counterion may be anionic or have a group with a net negative charge or charge polarization, cationic or have a group with a net positive charge or charge polarization, or zwitterionic and have a group with both negative and positive charges or charge polarization.

The terms "microparticle" and "microsphere" are interchangeable and refer to particles that comprise macromolecules and deliver an reagent of interest (such as a drug or nutritional supplement) to a subject to be treated, with a size range (average length, width, or diameter) of about or exactly 0.001 micrometers to about or exactly 500 micrometers. The

reagent may be a macromolecule, such as a protein, nucleic acid, lipid, or polysaccharide, or the macromolecule forming the microparticle may serve as a carrier for an active reagent, such as a drug or nutritional supplement. The microparticles may also comprise synthetic macromolecules, including polymers such as polyethylene glycol (PEG), polylactic acid (PLA), poly(lactic-co-glycolic acid) (PLGA), and natural polymers such as albumin, gelatin, chitosan, and dextran. The "microparticles" described herein may comprise and be prepared from a specific natural or synthetic macromolecule, or from more than one type of the same natural or synthetic macromolecule (e.g., more than one type of protein), or from a combination of more than one different type of natural or synthetic macromolecule (e.g., a protein and a nucleic acid, or a protein and a synthetic polymer).

As used herein, the term "microparticle" generally also refers to particles in a solid form that are not derived from the entire solution from which they are produced, although the particles formed by freezing and/or drying solutions containing macromolecules are also considered herein. Instead, the microparticles used herein are generally a collection of a portion of the solution components, including salts, counterions, solvents, and other ingredients, formed by methods including but not limited to precipitation, sedimentation, phase separation, and colloid formation.

The monoclonal antibody molecular size variant assay (CE-SDS) disclosed herein may adopt the capillary electrophoresis with sodium dodecyl sulfate (CE-SDS) using ultraviolet detection. Under reducing and non-reducing conditions, the purity of recombinant monoclonal antibody products is quantitatively determined according to molecular weight, in accordance with the method of Capillary Electrophoresis (General Chapter 0542, from: Pharmacopoeia of the People's Republic of China, 2015 Edition).

In one embodiment of the present disclosure, the glucocorticoid is conjugated to the N-terminal amino group and/or the ε-amino group of the lysine residue of the ligand via a linking unit. Generally, the number of drug molecules that can be conjugated to the antibody in the conjugation reaction will be less than the theoretical maximum.

The load of the antibody-drug conjugate can be controlled by the following non-limiting methods, including:

(1) controlling the molar ratio of the linking reagent to the monoclonal antibody,
(2) controlling the reaction time and temperature, and
(3) selecting different reaction reagents.

The term "amino acid" refers to an organic compound that contains both an amino group and a carboxyl group in its molecular structure, with both the amino group and the carboxyl group directly attached to the -CH- structure. The general formula is H2NCHRCOOH, where R is H, a substituted or unsubstituted alkyl group, or the like. According to the position of the amino group attached to the carbon atom in the carboxylic acid, amino acids can be classified into α-, β-, γ-, δ-, ε-... amino acids. In the biological world, the amino acids constituting natural proteins have specific structural characteristics, i.e., their amino groups are directly attached to the α-carbon atom, known as α-amino acids, including glycine, alanine, valine, leucine, isoleucine, phenylalanine, tryptophan, tyrosine, aspartic acid, histidine, asparagine, glutamic acid, lysine, glutamine, methionine, arginine, serine, threonine, cysteine, proline, and the like. Unnatural amino acids include, for example, citrulline. As commonly known to those skilled in the art, unnatural amino acids do not constitute natural proteins and thus do not participate in the synthesis of the antibodies in the present disclosure. The three-letter and single-letter amino acid codes used in the present disclosure are as described in J. biol. chem, 243, p3558 (1968).

The term "antibody" refers to an immunoglobulin, which is a tetrapeptide chain structure composed of two identical heavy chains and two identical light chains linked by interchain disulfide bonds. Due to differences in the amino acid composition and arranged sequence of the constant regions of immunoglobulin heavy chains, the antigenicity of the immunoglobulins also varies. Accordingly, immunoglobulins can be classified into five classes, also referred to as immunoglobulin isotypes, namely IgM, IgD, IgG, IgA, and IgE, with their corresponding heavy chains being μ chain, δ chain, γ chain, α chain, and ε chain, respectively. The same Ig class can be further divided into different subclasses based on differences in the amino acid composition of the hinge region and the number and position of heavy chain disulfide bonds. For example, IgG can be divided into IgG1, IgG2, IgG3, and IgG4. Light chains are classified into κ chain or λ chain based on differences in their constant regions. Each of the five Ig classes can have either κ chain or λ chain.

The sequences of approximately 110 amino acids near the N-terminus of both the heavy and light chains of an antibody vary significantly, which are referred to as a variable region (Fv region); and the remaining amino acid sequences near the C-terminus are relatively stable, known as a constant region. The variable region comprises 3 hypervariable regions (HVRs) and 4 framework regions (FRs) with relatively conserved sequences. The 3 hypervariable regions determine the specificity of the antibody and are also called complementarity-determining regions (CDRs). Each of the light chain variable region (LCVR) and the heavy chain variable region (HCVR) consists of 3 CDR regions and 4 FR regions, arranged sequentially in the following order from the amino terminus to the carboxyl terminus: FR1, CDRI1, FR2, CDR2, FR3, CDR3, and FR4. The 3 CDR regions of the light chain refer to LCDR1, LCDR2, and LCDR3; and the 3 CDR regions of the heavy chain refer to HCDR1, HCDR2, and HCDR3.

The antibodies of the present disclosure include murine-derived antibodies, chimeric antibodies, humanized antibodies, and fully human-derived antibodies, with humanized antibodies and fully human-derived antibodies being

preferred.

The term "murine-derived antibody" in the present disclosure refers to an antibody prepared using mice in accordance with the knowledge and skills in the art. During preparation, a specific antigen is injected into a test subject, followed by isolation of hybridomas that express antibodies with the desired sequences or functional characteristics.

The term "chimeric antibody" refers to an antibody formed by fusing a variable region of a murine-derived antibody with a constant region of a human antibody, which can reduce the immune response induced by the murine-derived antibody. To construct a chimeric antibody, first, a hybridoma secreting a murine-derived specific monoclonal antibody is established; then, the variable region gene is cloned from the murine hybridoma cells; the constant region gene of a human antibody is cloned as needed; the murine variable region gene and the human constant region gene are linked to form a chimeric gene, which is then inserted into an expression vector; and finally, the chimeric antibody molecule is expressed in a eukaryotic or prokaryotic system.

The term "humanized antibody", also known as a CDR-grafted antibody, refers to an antibody produced by grafting murine CDR sequences into variable region frameworks of a human antibody, i.e., framework sequences of different types of human germline antibodies. It can overcome the heterologous reaction induced by a large amount of murine protein components carried by chimeric antibodies. Such framework sequences can be obtained from public DNA databases containing germline antibody gene sequences or published references. For example, the germline DNA sequences of human heavy and light chain variable region genes can be found in the "VBase" human germline sequence database (available at internet: www.mrccpe.com.ac.uk/vbase) and in Kabat, E.A. et al., 1991, Sequences of Proteins of Immunological Interest, 5th edition. To avoid a decrease in activity while reducing immunogenicity, minimal back mutations or reverse mutations may be performed on the framework sequences of the human antibody variable regions to maintain activity. The humanized antibodies of the present disclosure also include those further subjected to affinity maturation of CDRs via phage display. Additional references describing methods for using mouse antibodies in humanization include, for example, Queen et al., Proc. Natl. Acad. Sci. USA, 88, 2869, 1991, and methods by Winter and colleagues [Jones et al., Nature, 321, 522 (1986); Riechmann et al., Nature, 332, 323-327 (1988); Verhoeyen et al., Science, 239, 1534 (1988)].

The term "fully human-derived antibody", "fully human antibody", or "completely human-derived antibody", also referred to as "fully human-derived monoclonal antibody", has both variable and constant regions of human origin, thereby eliminating immunogenicity and toxic side effects. The development of monoclonal antibodies has gone through four stages, namely: murine-derived monoclonal antibodies, chimeric monoclonal antibodies, humanized monoclonal antibodies, and fully human-derived monoclonal antibodies. The present disclosure relates to fully human-derived monoclonal antibodies. Relevant technologies for the preparation of fully human antibodies mainly include: human hybridoma technology, EBV-transformed B lymphocyte technology, phage display technology, transgenic mouse antibody preparation technology, and single B cell antibody preparation technology, etc.

The term "antigen-binding fragment" refers to one or more fragments of an antibody that retain the ability to specifically bind to an antigen. It has been shown that fragments of full-length antibodies can be used to perform the antigen-binding function of the antibodies. Examples of binding fragments contained in the "antigen-binding fragment" include (i) Fab fragments, i.e. monovalent fragments consisting of the VL, VH, CL, and CH1 domains; (ii) $F(ab')_2$ fragments, i.e. bivalent fragments comprising two Fab fragments linked by disulfide bridges on the hinge region; (iii) Fd fragments consisting of the VH and CH1 domains; (iv) Fv fragments consisting of the VH and VL domains of a single arm of an antibody; (v) single-domain or dAb fragments (Ward et al., (1989) Nature 341: 544-546), which consist of the VH domain; and (vi) isolated complementarity-determining regions (CDRs) or (vii) combinations of two or more isolated CDRs optionally linked by a synthetic linker. In addition, although the two domains, VL and VH, of the Fv fragment are encoded by separate genes, recombinant methods can be used to link them via a synthetic linker, thereby enabling the production of a single protein chain in which the VL and VH regions pair to form a monovalent molecule (referred to as single-chain Fv (scFv); see, e.g., Bird et al. (1988) Science 242: 423-426; and Huston et al. (1988) Proc. Natl. Acad. Sci. USA 85: 5879-5883). Such single-chain antibodies are also intended to be included in the term "antigen-binding fragment" of an antibody. Such antibody fragments are obtained using conventional techniques known to those skilled in the art and are screened for utility in the same manner as for intact antibodies. Antigen-binding moieties can be produced by recombinant DNA techniques or by enzymatic or chemical cleavage of intact immunoglobulins. Antibodies can be antibodies of different isotypes, e.g., IgG (e.g., IgG1, IgG2, IgG3, or IgG4 subtypes), IgA1, IgA2, IgD, IgE, or IgM antibodies.

Fab is an antibody fragment having a molecular weight of about 50,000 and having antigen binding activity in a fragment obtained by treating an IgG antibody molecule with the protease papain (cleaving the amino acid residue at position 224 of the H chain), in which about half of the N-terminal side of the H chain and the entire L chain bind to each other by disulfide bonds.

F(ab')2 is an antibody fragment with a molecular weight of about 100,000 and antigen-binding activity and comprising two Fab regions linked at the hinge position, obtained by digesting the lower part of the two disulfide bonds in the hinge region of an IgG with enzyme pepsin.

Fab' is an antibody fragment with a molecular weight of about 50,000 and antigen binding activity, obtained by cleaving the disulfide bonds in the hinge region of F(ab')2.

In addition, the Fab' can be produced by inserting the DNA encoding the Fab' fragment of an antibody into a prokaryotic or eukaryotic expression vector, followed by introducing the vector into a prokaryotic or eukaryotic organism for Fab' expression.

The term "single-chain antibody", "single-chain Fv" or "scFv" means a molecule comprising an antibody heavy chain variable domain (or region; VH) and an antibody light chain variable domain (or region; VL) linked by a linker. Such scFv molecules may have the general structure: $NH_2$-VL-linker-VH-COOH or $NH_2$-VH-linker-VL-COOH. Suitable prior art linkers consist of repeated GGGGS amino acid sequences or variants thereof, for example variants using 1-4 repeats (Holliger et al. (1993), Proc. Natl. Acad. Sci. USA90: 6444-6448). Other linkers useful in the present disclosure are described by Alfthan et al. (1995), Protein Eng.8:725-731, Choi et al. (2001), Eur.J.Immuno 1.31:94-106, Hu et al. (1996), Cancer Res.56:3055-3061, Kipriyanov et al. (1999), J.Mol.Biol.293:41-56 and Roovers et al. (2001), Cancer Immunol.

The term "CDR" refers to one of 6 hypervariable regions within variable domains of an antibody that primarily contribute to antigen binding. One of the most commonly used definitions for the 6 CDRs is provided by Kabat E.A. et al., (1991) Sequences of proteins of immunological interest. NIH Publication 91-3242). As used herein, the Kabat definition for CDRs applies only to CDR1, CDR2, and CDR3 of the light chain variable domain (CDR L1, CDR L2, CDR L3, or L1, L2, L3) and to CDR2 and CDR3 of the heavy chain variable domain (CDR H2, CDR H3, or H2, H3). In general, there are three CDRs (HCDR1, HCDR2, and HCDR3) in each heavy chain variable region, and there are three CDRs in each light chain variable region (LCDR1, LCDR2, and LCDR3). The amino acid sequence boundaries of CDRs can be determined using any of a variety of commonly known schemes, including the "Kabat" numbering rule (see Kabat et al. (1991), "Sequences of Proteins of Immunological Interest", 5th edition, Public Health Service, National Institutes of Health, Bethesda, MD), "Chothia" numbering rule (see Al-Lazikani et al., (1997) JMB 273:927-948) and ImMunoGenTics (IMGT) numbering rule (see Lefranc M. P., Immunologist, 7, 132-136 (1999); Lefranc, M.P. et al., Dev. Comp. Immunol., 27, 55-77 (2003)) and the like. For example, for the classical format, the CDR amino acid residues in the heavy chain variable domain (VH) are numbered as 31-35 (HCDR1), 50-65 (HCDR2), and 95-102 (HCDR3); and the CDR amino acid residues in the light chain variable domain (VL) are numbered as 24-34 (LCDR1), 50-56 (LCDR2), and 89-97 (LCDR3), following the Kabat rule. CDR amino acids in the VH are numbered as 26-32 (HCDR1), 52-56 (HCDR2), and 95-102 (HCDR3); and the amino acid residues in the VL are numbered as 26-32 (LCDR1), 50-52 (LCDR2), and 91-96 (LCDR3), following the Chothia rule. By combining the CDR definitions of both Kabat and Chothia, the CDR are composed of amino acid residues 26-35 (HCDR1), 50-65 (HCDR2), and 95-102 (HCDR3) in the human VH, as well as amino acid residues 24-34 (LCDR1), 50-56 (LCDR2), and 89-97 (LCDR3) in the human VL. Following the IMGT rule, CDR amino acid residues in the VH are approximately numbered as 26-35 (CDR1), 51-57 (CDR2), and 93-102 (CDR3), and CDR amino acid residues in the VL are approximately numbered as 27-32 (CDR1), 50-52 (CDR2), and 89-97 (CDR3). Following the IMGT rule, the CDR regions of an antibody can be determined using the program IMGT/DomainGap Align.

The term "antibody framework" refers to a portion of a variable domain VL or VH that serves as a scaffold of the antigen binding loop (CDR) of the variable domain. In essence, it is a variable domain with no CDR.

"Binding to IL-4R" refers to the ability to interact with human IL-4R (or an epitope, fragment thereof). The term "antigen-binding site" herein refers to a three-dimensional spatial site recognized by the antibody or antigen-binding fragment herein.

The term "epitope" or "antigenic determinant" refers to the site on an antigen to which an immunoglobulin or antibody specifically binds. Epitopes generally include at least 3, 4, 5, 6, 7, 8, 9, 10, 11, 12, 13, 14, or 15 contiguous or non-contiguous amino acids in a unique spatial conformation (see, e.g., Epitope Mapping Protocols in Methods in Molecular Biology, vol. 66, G.E.Morris, Ed. (1996)).

The terms "specifically binds", "selectively binds", "selectively binds" and "specifically binds" refer to the binding of an antibody to a predetermined epitope on an antigen. Typically, the antibody binds with an affinity (KD) of approximately less than $10^{-7}$ M, for example, approximately less than $10^{-8}$ M, $10^{-9}$ M, $10^{-10}$ M, or lower.

The term "nucleic acid molecule" refers to DNA molecules and RNA molecules. Nucleic acid molecules can be single-stranded or double-stranded, but are preferably double-stranded DNAs. When a nucleic acid is placed in a functional relationship with another nucleic acid sequence, the nucleic acids are "operably linked". For example, if a promoter or enhancer affects the transcription of a coding sequence, the promoter or enhancer is operably linked to the coding sequence.

The term "vector" refers to a nucleic acid molecule that is capable of transporting another nucleic acid to which it is attached. In one embodiment, a vector is a "plasmid", which refers to a circular double-stranded DNA loop into which an additional DNA segment can be ligated. In another embodiment, the vector is a viral vector in which an additional DNA segment can be ligated into the viral genome. The vectors disclosed herein can undergo autonomous replication in the host cells into which they have been introduced (e.g., bacterial vectors with a bacterial origin of replication and episomal mammalian vectors) or can integrate into the genome of the host cell upon introduction into the host cell, thereby replicating along with the host genome (e.g., non-episomal mammalian vectors).

Methods for producing and purifying antibodies and antigen-binding fragments are well known in the prior art, such as Chapters 5-8 and 15 of the Antibody Experimental Technology Guide, Cold Spring Harbor. Antigen-binding

fragments can also be prepared by conventional methods. The antibodies or antigen-binding fragments described in the invention are produced by genetic engineering methods, wherein one or more human-derived FR regions are added to the non-human-derived CDR regions. Human FR germline sequences can be obtained by aligning with the IMGT human antibody variable region germline gene database and MOE software, from the ImMunoGeneTics (IMGT) website, http://imgt.cines.fr, or from the Journal of Immunoglobulins (2001ISBN012441351).

The term "host cell" refers to a cell into which an expression vector has been introduced. Host cells may include bacterial, microbial, plant, or animal cells. Bacteria susceptible to transformation include members of enterobacteriaceae, such as strains of *Escherichia coli* or *Salmonella;* Bacillaceae such as *Bacillus subtilis;* Pneumococcus; Streptococcus; and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

Engineered antibodies or antigen-binding fragments of the present disclosure can be prepared and purified by conventional methods. For example, cDNA sequences encoding heavy and light chains can be cloned and recombined into GS expression vectors. CHO cells can be stably transfected with recombinant immunoglobulin expression vectors. As a more recommended prior art, mammalian expression systems may result in glycosylation of antibodies, particularly at the highly conserved N-terminal sites in the Fc region. Positive clones are subjected to expanded culture in serum-free media in bioreactors to produce antibodies. The culture solution containing secreted antibodies can be purified using conventional techniques. For example, it is purified using an A or G Sepharose FF column containing an adjusted buffer. Non-specifically bound components are washed off. Bound antibodies are eluted by a pH gradient method, and antibody fragments are detected by SDS-PAGE and collected. Antibodies can be concentrated by filtration using conventional methods. Soluble mixtures and multimers can also be removed by conventional methods, such as molecular sieves, and ion exchange. The resulting products should be frozen immediately, e.g. at -70°C, or lyophilized.

The "identity" of amino acid sequences refers to the percentage of amino acid residues in a first sequence that are identical to those in a second sequence, after aligning the amino acid sequences and introducing gaps as necessary to achieve the maximum sequence identity percentage, with no conservative substitution considered as part of sequence identity. The alignment for purposes of determining percent amino acid sequence identity can be achieved in a variety of ways within the scope of the skill in the art, for example using publicly available computer softwares, such as BLAST, BLAST-2, ALIGN, ALIGN-2 or Megalign (DNASTAR) software. Those skilled in the art can determine parameters suitable for measuring alignment, including any algorithm needed to achieve the maximum alignment over the full length of the sequences being compared.

The term "anti-IL-4R antibody" or "an antibody that binds to IL-4R" refers to an antibody capable of binding to IL-4R with, for example, sufficient affinity, so that the antibody can be used as a therapeutic agent targeting IL-4R. The binding agree of an anti-IL-4R antibody to an unrelated non-IL-4R protein may be less than about 10% of the antibody's binding to IL-4R as measured, for instance, by radioimmunoassay (RIA). In some embodiments, the antibody that binds to IL-4R has a dissociation constant (Kd) of $\leq$ 1 $\mu$M, $\leq$ 100 nM, $\leq$ 10 nM, $\leq$ 1 nM or $\leq$ 0.1 nM.

The term "peptide" refers to a compound fragment between amino acids and proteins, formed by 2 or more than 2 amino acid molecules linked to each other through peptide bonds, and is a structural and functional fragment of proteins. Substances such as hormones and enzymes are essentially peptides.

The term "sugar" refers to a biological macromolecule consisting of three elements, C, H and O, which can be divided into monosaccharides, disaccharides, polysaccharides, etc.

The term "pharmaceutical composition" denotes a mixture containing one or more antibody-drug conjugates described herein or physiologically/pharmaceutically acceptable salts or prodrugs thereof and other chemical components, such as physiologically/pharmaceutically acceptable carriers and excipients. The purpose of the pharmaceutical composition is to maintain the stability of the antibody active ingredient, and to facilitate administration to living organisms and facilitate the absorption of active ingredients to exert biological activity.

In the present disclosure, "pharmaceutical compositions" and "preparations" are not mutually exclusive.

For the solution form of the pharmaceutical composition described in the present disclosure, unless otherwise specified, the solvent therein is water.

"Lyophilized preparation" refers to a preparation or pharmaceutical composition obtained by subjecting a pharmaceutical composition in a liquid or solution form or a liquid or solution preparation to a vacuum freeze-drying step.

As used herein, the term "about" or "approximately" means that the numerical value is within an acceptable error range for a particular value as determined by a person of ordinary skill in the art, which will depend in part on how the value is measured or determined (i.e., the limitations of the measurement system). For example, "about" may mean within 1 or more than 1 standard deviation, per the practice in the art. Alternatively, "about" or "substantially comprising" may mean a range of up to 20%. In addition, particularly with respect to biological systems or processes, the term may mean within at most an order of magnitude or within at most 5-fold of a value. Unless otherwise stated, when a particular value appears in the present application and claims, the meaning of "about" or "substantially comprising" shall be assumed to be within an acceptable error range for that particular value.

The numerical values in the present disclosure are measured values from instruments or calculated values

derived from instrument measurements, which have a certain degree of error. In general, plus or minus 10% is within a reasonable error range. Of course, the context in which the numerical value is used needs to be considered. For example, with regard to the content of total impurities, the change in the error of the numerical value after measurement does not exceed plus or minus 10%, and may be plus or minus 9%, plus or minus 8%, plus or minus 7%, plus or minus 6%, plus or minus 5%, plus or minus 4%, plus or minus 3%, plus or minus 2% or plus or minus 1%, preferably plus or minus 5%.

"Optional" or "optionally" means that the event or circumstance subsequently described may but need not occur, and the description includes the case where the event or circumstance occurs or does not occur. For example, "optionally comprising 1-3 antibody heavy chain variable regions" means that the antibody heavy chain variable regions of particular sequences may exist, but are not required to exist.

The term "substituted" refers to one or more hydrogen atoms in the group, preferably at most 5, more preferably 1 to 3 hydrogen atoms each independently substituted with a corresponding number of substituents. It goes without saying, the substituents may be only in their possible chemical positions, a person skilled in the art can determine the possible or impossible substitutions (by experiment or theory) without paying too much effort. For example, an amino group or hydroxyl group with a free hydrogen may be unstable when it binds to a carbon atom having an unsaturated (e.g., olefinic) bond.

For the preparation of conventional pharmaceutical compositions, reference can be made to the Chinese Pharmacopoeia.

When applied to animals, humans, experimental subjects, cells, tissues, organs, or biological fluids, the terms "administering" and "treating" refer to the contact of an exogenous drug, therapeutic agent, diagnostic agent, or composition with an animal, human, subject, cell, tissue, organ, or biological fluid. "Administering" and "treating" may refer to, for example, therapeutic, pharmacokinetic, diagnostic, research, and experimental methods. Treating cells includes contact of a reagent with the cells, and contact of a reagent with a fluid, wherein the fluid is in contact with the cells. "Administering" and "treating" also mean treating, for example, cells in vitro and ex vivo by reagents, diagnostic agents or binding compositions or by another type of cell. "Treating", when applied to a human, veterinary or study subject, refers to a therapeutic treatment, prevention or prophylactic measure, research and diagnostic applications.

"Treatment" refers to the internal or external administration of an therapeutic agent to a patient, for example, a composition comprising any of the binding compounds of the present disclosure, wherein the patient has one or more disease symptoms, and the therapeutic agent is known to have a therapeutic effect on such symptoms. Typically, the therapeutic agent is administered in an amount effective to alleviate one or more disease symptoms in the treated patient or population, so as to induce the regression of such symptoms or inhibit the progression of such symptoms to any clinically measurable extent. The amount of a therapeutic agent effective to alleviate any particular disease symptom (also known as a "therapeutically effective amount") can vary depending on a variety of factors, such as the patient's disease state, age and body weight, and the ability of the drug to produce the desired therapeutic effect in the patient. Whether disease symptoms have been alleviated can be assessed by any clinical detection method usually used by physicians or other healthcare professionals to evaluate the severity or progression of the symptoms. Although the embodiments of the present disclosure (e.g., treatment methods or articles of manufacture) may not be effective in alleviating every target disease symptom, they should reduce the target disease symptoms in a statistically significant number of patients, as determined by any statistical test method known in the art, such as Student's t-test, chi-square test, Mann-Whitney U test, Kruskal-Wallis test (H test), Jonckheere-Terpstra test, and Wilcoxon test.

The "effective amount" includes an amount sufficient to ameliorate or prevent a symptom or condition of a medical disease. The effective amount also means an amount sufficient to allow or facilitate diagnosis. The effective amount for a particular patient or veterinary subject may vary depending on the following factors: for example, the condition to be treated, the patient's general health, the method, route and dosage of administration, and the severity of side effects. The effective amount may be the maximum dosage or administration scheme that avoids significant side effects or toxic effects.

"Exchange" refers to the process of exchanging the solvent system in which an antibody protein or antibody-drug conjugate is dissolved. For example, through a physical operation, a high-salt or hypertonic solvent system containing the antibody protein or antibody-drug conjugate is exchanged with a buffer system for stabilizing a preparation, thereby resulting in the antibody protein or antibody-drug conjugate being present in the stable preparation. The physical operation includes, but is not limited to, ultrafiltration, dialysis, or reconstitution after centrifugation.

## Detailed Description of Embodiments

The present disclosure will be further described below in conjunction with examples, but these examples are not intended to limit the scope of the present disclosure. Experimental methods for which specific conditions are not specified in the embodiments of the present disclosure, are performed generally under conventional conditions, or under conditions recommended by the manufacturer of starting materials or goods, for example, referring to the Antibody Technology Laboratory Manual or Molecular Cloning: A Laboratory Manual, published by Cold Spring Harbor Laboratory; or according to the conditions recommended by the manufacturer of starting materials or goods. The reagents for which the specific

source is not indicated, are conventional reagents that are commercially available.

The NMR shift (δ) is given in the unit of $10^{-6}$ (ppm). NMR assay is performed using Bruker AVANCE-400 nuclear magnetic resonance spectrometer; the solvents for determination are deuterated dimethyl sulfoxide (DMSO-$d^6$), deuterated chloroform ($CDCl_3$) and deuterated methanol ($CD_3OD$); and the internal standard is tetramethylsilane (TMS).

The MS assay is performed using Shimadzu 2010 mass spectrometer or Agilent 6110A MSD mass spectrometer.

The HPLC assay is performed using Shimadzu LC-20A systems, Shimadzu LC-2010HT series or Agilent 1200 LC high-pressure liquid chromatograph (Ultimate XB-C18 3.0*150 mm chromatography column or Xtimate C18 2.1*30 mm chromatography column).

Chiral HPLC analysis assay is performed using Chiralpak IC-3 100 × 4.6 mm I.D., 3 um; Chiralpak AD-3 150 × 4.6 mm I.D., 3 um; Chiralpak AD-3 50 × 4.6 mm I.D., 3 um; Chiralpak AS-3 150 × 4.6 mm I.D., 3 um; Chiralpak AS-3 100 × 4.6 mm I.D., 3 μm; ChiralCel OD-3 150 × 4.6 mm I.D., 3 um; Chiralcel OD-3 100 × 4.6 mm I.D., 3 μm; ChiralCel OJ-H 150 × 4.6 mm I.D., 5 um; and Chiralcel OJ-3 150 × 4.6 mm I.D., 3 um chromatography column. The silica gel plate for thin layer chromatography is Yantai Huanghai HSGF254 or Qingdao GF254 silica gel plate, the specification of the silica gel plate used for thin layer chromatography (TLC) is 0.15 mm to 0.2 mm, and the specification of the silica gel plate used for separating and purifying products by thin layer chromatography is 0.4 mm to 0.5 mm.

For column chromatography, Yantai Huanghai silica gel with 100-200 mesh, 200-300 mesh or 300-400 mesh is generally used as a carrier.

For chiral preparative column, DAICEL CHIRALPAK IC (250 mm * 30 mm, 10 um) or Phenomenex-Amylose-1 (250 mm * 30 mm, 5 um) is used.

The CombiFlash flash preparative instrument used is Combiflash Rf150 (TELEDYNE ISCO).

The determination of the average kinase inhibition rate and $IC_{50}$ value is performed using NovoStar microplate reader (BMG Company, Germany).

Known starting materials in the present disclosure may be synthesized using or according to methods known in the art, or may be purchased from ABCR GmbH & Co. KG, Acros Organics, Aldrich Chemical Company, Accela ChemBio Inc., Chembee Chemicals, and other companies.

If there is no special instruction in the examples, reactions can all be carried out under argon atmosphere or nitrogen atmosphere.

The argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

Parr 3916EKX hydrogenator, Qinglan QL-500 hydrogen generator or HC2-SS hydrogenator are used for pressurized hydrogenation reaction.

The hydrogenation reaction usually comprises the steps of vacuumizing and charging with hydrogen, and the operation is repeated for 3 times.

CEM Discover-S 908860 microwave reactor is used for the microwave reaction.

Unless otherwise specified in the examples, a solution refers to an aqueous solution.

Unless otherwise indicated, the reaction temperature in the examples refers to room temperature, which is 20°C to 30°C.

The progress of the reaction in the examples is monitored by means of thin layer chromatography (TLC). The developing solvent used in the reaction, the eluent system of column chromatography used in the purification of compounds and the developing solvent system of thin layer chromatography include: A: dichloromethane/methanol system, B: n-hexane/ethyl acetate system, C: petroleum ether/ethyl acetate system, D: petroleum ether/ethyl acetate/-methanol, wherein the volume ratio of the solvent is adjusted according to the polarity of compounds and can also be adjusted by adding a small quantity of basic reagents such as triethylamine or acidic reagents such as acetic acid.

Formulation of 1.0 M Tris buffer, pH = 8.30 ± 0.1:
6.0 g of tris was weighed into a 50 mL volumetric flask, and 40 mL of purified water was added, to allow the substance to be dissolved by shaking. 1.2 mL of concentrated hydrochloric acid was added dropwise to adjust the pH to 8.30, and then purified water was added to make up to the mark.

Formulation of Buffer A:
In a 2.0 L container, $KH_2PO4$ (8.50 g), $K_2HPO4$ (8.56 g), NaCl (5.86 g), and EDTA (1.50 g) were added. Then 1.6 L of water for injection was added, and stirred for half an hour to allow the mixture to be dissolved completely, then water for injection was added to make up the volume to 2.0 L, and the pH was measured as 6.30 ± 0.1.

The abbreviations used in the following experiments have the following meanings:
DAST: diethylaminosulfur trifluoride; THF: tetrahydrofuran; NMP: *N*-methyl pyrrolidone; DCM: dichloromethane; *m*-CPBA: m-chloroperoxybenzoic acid; DIEA: *N,N*-diisopropylethylamine; TEA: triethylamine; Boc: tert-butoxycarbonyl, MeOH: methanol; $Et_2O$: diethyl ether.

The preparation and purification methods of antibodies 25G7 and 7B10 in the present application have been

recorded in the patent document with application number WO 2020038454 A1, and the preparation methods of 25G7-Fab and ADC have been recorded in the patent document with application number PCT/CN2023/073057. The entire contents of the aforementioned application documents are incorporated herein.

**Example 1: Preparation of N-((10*S*)-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d][1,3]dioxol-8b-yl)-1,6,9,12,15-pentaoxa-3-oxo-5,8,11,14-tetraethylene glycol-16-yl)-1-(2-bromoacetamido)-3,6,9,12-tetraoxapentadecane-15-amide** (compound 1)

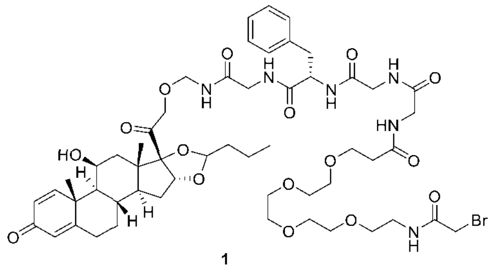

1

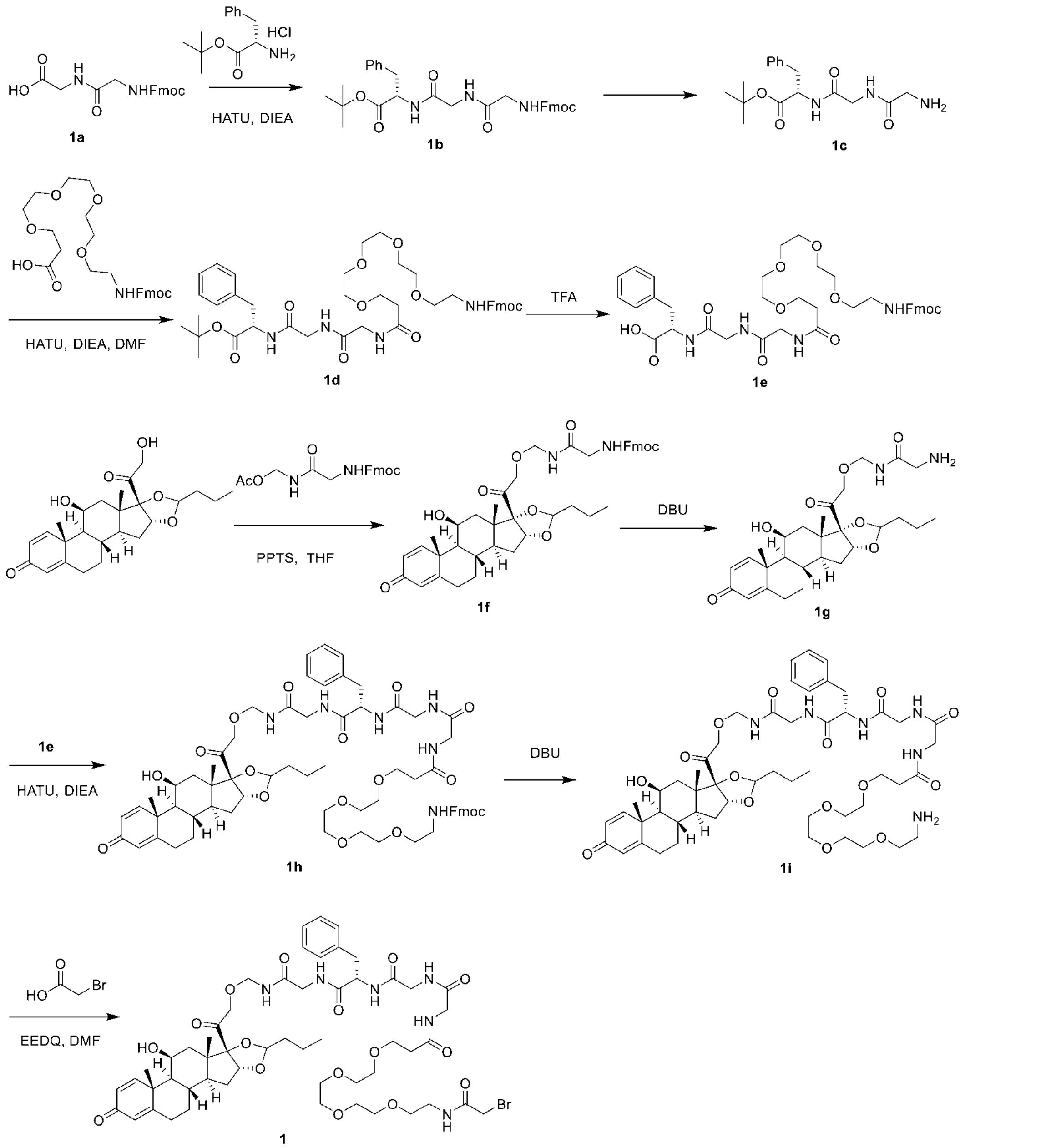


**Step 1) Preparation of tert-butyl(((9*H*-fluoren-9-yl)methoxy)carbonyl)glycylglycyl-L-phenylalaninate** (compound 1b)

Compound **1a** (5.14 g, 20.0 mmol, 1.0 eq) and L-phenylalanine tert-butyl ester hydrochloride (7.08 g, 20.0 mmol, 1.0 eq) were placed in a 250 mL three-necked flask, and dissolved in anhydrous DMF (60 mL) to give a clear solution. The mixture was cooled in an ice bath, then HATU (9.12 g, 24.0 mmol, 1.2 eq) and DIEA (7.74 g, 60.0 mmol, 3.0 eq) were added, and the resulting mixture was maintained in an ice bath and reacted for 2 hours. Water was added to the reaction solution, the mixture was extracted with ethyl acetate, and the organic phase was washed with a saturated aqueous sodium chloride solution, dried, and concentrated under reduced pressure to obtain compound **1b** (11.5 g, 100% yield). The crude product can be directly used in the next step.

MS (ESI): m/z 580.3 $[M+Na]^+$.

$^1H$ NMR (400 MHz, DMSO-d6) $\delta$ 8.21-8.16(m, 1H), 8.12-8.05 (m, 1H), 7.91-7.87 (m, 2H), 7.72-7.68 (m, 2H), 7.64-7.59 (m, 1H), 7.44-7.38 (m, 2H), 7.36-7.25 (m, 4H), 7.23-7.18 (m, 3H), 4.41-4.18 (m, 4H), 3.76-3.72 (m, 2H), 3.68-3.61 (m, 2H), 2.98-2.89 (m, 2H), 2.69 (s, 2H), 1.30 (s, 9H).

**Step 2) Preparation of glycylglycyl-L-phenylalaninate tert-butyl ester** (compound 1c)

Compound 1b (5.57 g, 10.0 mmol, 1.0 eq) was placed in a 250 mL three-necked flask, dissolved in anhydrous DCM (60 mL) to give a clear solution and cooled in an ice bath. Piperidine (8.5 g, 100.0 mmol, 10.0 eq) was added slowly, and after the addition was complete, the reaction mixture was stirred at room temperature for about 2 hours. The reaction solution was directly concentrated to dryness, and the crude product was purified by column chromatography to obtain compound **1c** (2.70 g, 81% yield).
MS (ESI): m/z 358.3$[M+H]^+$.

**Step 3) Preparation of (1-(9*H*-fluoren-9-yl)-3-oxo-2,7,10,13,16-pentaoxa-4-tetraethylene glycol-19-oxy)glycylglycyl-L-phenylalaninate tert-butyl** ester(compound 1d)

Starting materials 1-(9H-fluoren-9-yl)-3-oxo-2,7,10,13,16-pentaoxa-4-tetraethylene glycol-19-oleic acid (1.0 g, 2.05 mmol, 1.0 eq; sourced from Tonglai Biochemical) and **1c** (0.69 g, 2.05 mmol, 1.0 eq) to a 100 mL three-necked flask, dissolved in anhydrous DMF (25 mL) to give a clear solution, and cooled in an ice bath. HATU (1.01 g, 2.67 mmol, 1.3 eq) and DIEA (529 mg, 4.10 mmol, 2.0 eq) were added and the ice bath was maintained for reaction for 1 hours. Water was added to the reaction solution, the mixture was extracted with ethyl acetate, washed with a saturated aqueous sodium chloride solution, dried, and concentrated under reduced pressure to obtain compound **1d** (1.60 g, 97% yield).
MS (ESI): m/z 805.3 $[M+H]^+$.

**Step 4) Preparation of (1-(9*H*-fluoren-9-yl)-3-oxo-2,7,10,13,16-pentaoxa-4-tetraethylene glycol-19-oxy)glycylglycyl-L-phenylalanine** (compound 1e)

Compound **1d** (1.60 g, 1.99 mmol, 1.0 eq) was added to a 100 mL single-necked flask, and dissolved by addition of anhydrous dichloromethane (16 mL). Trifluoroacetic acid (8 mL) was added at room temperature, and the stirring was maintained for reaction for 2 hours. The reaction solution was directly concentrated to dryness, dissolved by addition of ethyl acetate, washed with a saturated aqueous sodium chloride solution, dried, and concentrated under reduced pressure to obtain compound **1e** (1.23 g, 83% yield).
MS (ESI): m/z 749.3 $[M+H]^+$.

**Step 5) Preparation of (9*H*-fluoren-9-yl)methyl(2-(((2-((6aR,6bS,7S,8aS,8bS,11aR,12aS,12bS)-7-hydroxy-6a,8a-dimethyl-4-oxo-10-propyl-1,2,4,6a,6b,7,8,8a,11a,12,12a,12b-dodecahydro-8bH-naphtho[2',1':4,5]indeno[1,2-d][1,3]dioxol-8b-yl)-2-oxoethoxy)methyl)amino)-2-oxoethyl)carbamate** (compound 1f)

Starting materials budesonide (1.29 g, 3.0 mmol, 1.0 eq), methyl(2-((((9H-fluoren-9-yl)methoxy)carbonyl) amino)acetamido)acetate (1.16 g, 3.15 mmol, 1.0 eq; prepared according to the method in the reference "Tetrahedron, 2018, 74(15), 1951-1956"), and pyridinium p-toluenesulfonate (75 mg, 0.30 mmol, 0.1 eq) were added to a 100 mL three-necked flask. Anhydrous tetrahydrofuran (20 mL) was added at room temperature, and the mixture was heated under reflux and reacted for 4 hours. The reaction solution was directly concentrated to dryness, and the crude product was purified by column chromatography to obtain **1f** (0.54 g, 24% yield).
MS (ESI): m/z 739.4 $[M+H]^+$.
$^1H$ NMR (400 MHz, DMSO-d6) $\delta$ 8.76-8.69 (m, 1H), 7.91-7.84 (m, 2H), 7.73-7.67 (m, 2H), 7.63-7.57 (m, 1H), 7.43-7.58 (m, 2H), 7.35-7.23 (m, 3H), 6.13 (d, *J* = 7.5 Hz, 1H), 5.91 (s, 2H), 5.15-4.97 (m, 1H), 4.73-4.45 (m, 5H), 4.30-4.09 (m, 5H), 3.65-3.59 (m, 2H), 2.29-2.21 (m, 1H), 2.11-1.87 (m, 2H), 1.74-1.21 (m, 11H), 1.09-0.77 (m, 8H).

**Step 6) preparation of 2-amino-N-((2-((6a*R*,6b*S*,7*S*,8a*S*,8b*S*,11a*R*,12a*S*, 12b*S*)-7-hydroxy-6a,8a-dimethyl-4-oxo-10-propyl-1,2,4,6a,6b,7,8,8a, 11a,12,12a,12b-dodecahydro-8bH-naphtho[2',1':4,5]indeno[1,2-d][1,3]dioxol-8b-yl)-2-oxoethoxy)methyl)acetamide** (compound 1g)

Compound 1f (540 mg, 0.73 mmol, 1.0 eq) was placed in a 25 mL single-necked flask, and dissolved by addition of anhydrous dichloromethane (10 mL). The mixture was cooled in an ice bath, DBU (167 mg, 1.10 mmol, 1.5 eq) was added and the stirring was maintained for reaction for 30 minutes. Water was added for liquid separation, the aqueous phase was extracted with dichloromethane twice, washed with a saturated aqueous sodium chloride solution, and concentrated under reduced pressure to obtain compound **1g** (450 mg, 100% yield). The crude product can be directly used in the next reaction.
MS (ESI): m/z 517.4 $[M+H]^+$.

**Step 7) Preparation of (9*H*-fluoren-9-yl)methyl ((10*S*)-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho [2',1':4,5]indeno[1,2-d][1,3]dioxol-8b-yl)-1,6,9,12,15,18-hexaoxa-3,21,24,27,30-pentaoxo-5,8,11,14,17-pentaazatriacontan-32-yl)carbamate** (compound 1h)

**Compounds 1g** (0.45 g crude product, converted to 0.73 mmol, 1.0 eq) and 1e (0.55 g, 0.73 mmol, 1.0 eq) were placed into a 100 mL three-necked flask, and dissolved in anhydrous DMF (9 mL) to give a clear solution. The mixture was cooled in an ice bath, then HATU (361 mg, 0.95 mmol, 1.3 eq) and DIEA (189 mg, 1.46 mmol, 2.0 eq) were sequentially added, and the stirring was maintained for reaction for 1 hour. Water was added to the reaction solution, the mixture was extracted with dichloromethane twice, and the organic phases were combined, washed once with a saturated aqueous sodium chloride solution, dried and concentrated under reduced pressure to obtain a crude product. The crude product was purified by slurrying in mixed solvents of petroleum ether and ethyl acetate to obtain compound **1h** (710 mg, 78% yield).

MS (ESI): m/z 1269.7 $[M+Na]^+$.

**Step 8) Preparation of 1-amino-*N*-((10*S*)-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d] [1,3] dioxol-8b-yl)-1,6,9,12,15-pentaoxa-3-oxo-5,8,11,14-tetraethylene glycol-16-yl-3,6,9,12-tetraoxapentadecane-15-amide** (compound 1i)

Compound **1h** (350 mg, 0.28 mmol, 1.0 eq) was placed into a 25 mL single-necked flask, and dissolved in anhydrous dichloromethane (10 mL) to give a clear solution. The mixture was cooled in an ice bath, DBU (64 mg, 0.42 mmol, 1.5 eq) was added and the stirring was maintained for reaction for 1 hour. The reaction solution was directly concentrated to dryness to obtain a crude product, which was further purified by column chromatography to obtain compound 1i (289 mg, 89% yield).

MS (ESI): m/z 1025.6 $[M+H]^+$.

**Step 9) Preparation of N-((10*S)*-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d] [1,3]dioxol-8b-yl)-1,6,9,12,15-pentaoxa-3-oxo-5,8,11,14-tetraethylene glycol-16-yl)-1-(2-bromoacetamido)-3,6,9,12-tetraoxapentadecane-15-amide** (compound 1)

Solid bromoacetic acid (35.2 mg, 0.127 mmol, 1.0 eq) and EEDQ (63 mg, 0.25 mmol, 2.0 eq) were placed into a 25 ml Schlenk-Tube, and dissolved in anhydrous DMF (5 mL) to give a clear solution. The reaction was stirred at room temperature for 1 hour. Compound **1i** (130 mg, 0.127 mmol, 1.0 eq) was dissolved in DMF (2 mL), this solution was added dropwise to the active ester solution prepared above, and after completion of addition, the reaction was maintained at room temperature for another 2 hours. Water was added to the reaction system, the mixture was extracted with ethyl acetate twice, and the organic phases were combined, washed with a saturated aqueous sodium chloride solution, dried and concentrated under reduced pressure to obtain a crude product, which was purified by column chromatography to obtain compound 1 (39 mg, 27% yield).

MS (ESI): m/z 1145.5/1147.6 $[M+1]^+$.

$^1$H NMR (400 MHz, $CDCl_3$) $\delta$ 8.62-8.57 (m, 1H), 8.34-8.28 (m, 2H), 8.19-8.11(m, 2H), 8.03-7.99(m, 1H), 7.30-7.17(m, 6H), 6.15 (d, *J* = 9.6 Hz, 1H), 5.92 (s, 2H), 5.18-5.03 (m, 1H), 4.72-4.47 (m, 5H), 4.29-4.15 (m, 2H), 3.78-3.42 (m, 27H), 3.27-3.21 (m, 2H), 3.06-2.84 (m, 2H), 2.41-2.28 (m, 3H), 2.06-1.73 (m, 2H), 1.60-1.24 (m, 10H), 1.01-0.82 (m, 8H).

**Example 2:**

**N-((10*S*)-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d][1,3]dioxol-8b-yl)-1,6,9,12,15-pentaoxa-3-oxo-5,8,11,14-tetraethylene glycol-16-yl)-1-(2,5-dioxo-2,5-dihydro-1*H*-pyrrol-1-yl)-3,6,9,12-tetraoxapentadecane-15-amide**

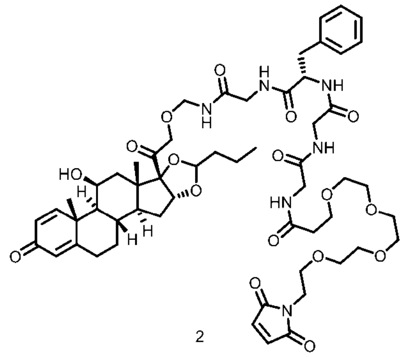

2

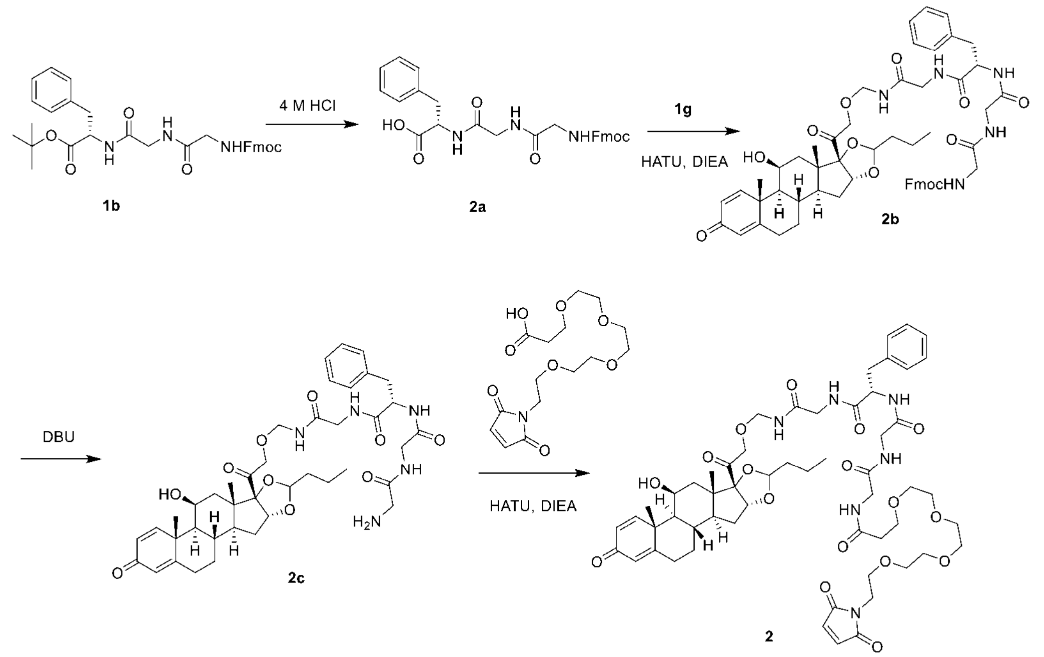


**Step 1) Preparation of (((9*H*-fluoren-9-yl)methoxy)carbonyl)glycylglycyl-L-phenylalanine** (compound 2a)

Compound **1b** (1.50 g, 2.69 mmol, 1.0 eq) was placed into a 100 mL single-necked flask, and dissolved by addition of a 4 M HCl solution in 1,4-dioxane (20 mL). Stirring was maintained for 4 hours at room temperature until the reaction of starting material **1b** was complete. The reaction solution was directly concentrated to dryness to obtain compound **1a** (1.34 g, 100% yield).
MS (ESI): m/z 502.3 $[M+1]^+$

**Step 2) Preparation of (9*H*-fluoren-9-yl)methyl-((10*S*-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d][1,3]dioxol-8b-yl)-1,6,9,12,15-pentaoxa-3-oxo-5,8,11,14-tetraethylene glycol-16-yl)carbamate** (compound 2b)

Compounds **2a** (0.97 g, 1.63 mmol, 1.0 eq) and **1g** (1.0 g, 1.95 mmol, 1.2 eq) were placed in a 100 mL three-necked flask. DMF (20 mL) was added and the mixture was cooled in an ice bath. HATU (927 mg, 2.44 mmol, 1.5 eq) and DIEA (420 mg, 3.26 mmol, 2.0 eq) were added sequentially, and after completion of addition, the ice bath was maintained for reaction for 1-2 hours. The reaction solution was concentrated, and purified by column chromatography to obtain compound **2b** (860 mg, 53% yield).
MS (ESI): m/z 1000.5 $[M+1]^+$

**Step 3 Preparation of (2*S*)-2-(2-(2-aminoacetamido)acetamido)-*N*-(2-(((2-((6a*R*,6b*S*,7*S*,8a*S*,8b-*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d][1,3]dioxol-8b-yl)-2-oxoethoxy)methyl)amino)-2-oxoethyl)-3-phenylpropanamide** (compound 2c)

Compound **2b** (860 mg, 0.86 mmol, 1.0 eq) was placed in a 25 mL single-necked flask, and dissolved in anhydrous DMF (10 mL) to give a clear solution. The mixture was cooled in an ice bath, DBU (196 mg, 1.29 mmol, 1.5 eq) was added and the stirring was maintained for reaction for 30 minutes. The reaction solution was directly concentrated, and purified by column chromatography to obtain compound **2c** (485 mg, 72% yield).
MS (ESI): m/z 778.4 $[M+1]^+$

**Step 4) Preparation of *N*-((10*S*)-10-benzyl-1-((6a*R*,6b*S*,7*S*,8a*S*,8b*S*,11a*R*, 12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d][1,3]dioxol-8b-yl)-1,6,9,12,15-pentaoxa-3-oxo-5,8,11,14-tetraethylene glycol-16-yl)-1-(2,5-dioxo-2,5-dihydro-1*H*-pyrrol-1-yl)-3,6,9,12-tetraoxapentadecane-15-amide** (compound 2)

Compound **2c** (480 mg, 0.62 mmol, 1.0 eq) and 1-(2,5-dioxo-2,5-dihydro-1H-pyrrol-1-yl)-3,6,9,12-tetraoxapentadecan-15-oic acid (213 mg, 0.62 mmol, 1.0 eq.; sourced from Tonglai Biochemical) were placed into a 100 mL three-necked flask, and dissolved in anhydrous DMF (10 mL) to give a clear solution. The mixture was cooled in an ice bath, then HATU (352 mg, 0.93 mmol, 1.5 eq) and DIEA (159 mg, 1.23 mmol, 2.0 eq) were sequentially added, and the stirring was maintained for reaction for 1 hour. The reaction solution was directly concentrated, and purified by column chromatography to obtain compound 2 (360 mg, 53% yield).

MS (ESI): m/z 1105.6 $[M+1]^+$
$^1$H NMR (400 MHz, DMSO-d6) $\delta$ 8.61-8.56 (m, 1H), 8.32-8.26 (m, 1H), 8.22-8.13 (m, 2H), 8.03-7.96(m, 1H), 7.39-7.20(m, 6H), 7.02 (s, 2H), 6.17-6.13 (m, 1H), 5.92 (s, 2H), 5.16-4.99 (m, 1H), 4.76-4.40 (m, 6H), 4.29-4.16 (m, 2H), 3.64-3.19 (m, 26H), 3.09-3.02 (m, 1H), 2.86-2.79 (m, 1H), 2.41-2.26 (m, 3H), 2.12-1.90 (m, 2H), 1.78-1.25 (m, 10H), 1.03-0.83 (m, 8H).

## Example 3: Preparation process of 25G7-Fab protein

The gene sequence of the antibody was synthesized and subcloned into the pcDNA3.4 vector. The ligation product was transformed into Top10 competent cells, and positive clones were picked for expansion culture. The clones were inoculated into the medium for expansion culture, followed by large-scale extraction of the plasmid containing the deoxyribonucleic acid sequence of the 25G7-Fab antibody. Lipofection was used, during which the expression plasmid was mixed with the transfection reagent, then added to Expi 293 cells, and the cell culture was harvested after 5 days of incubation in a constant-temperature incubator. The cell culture was centrifuged to collect the supernatant, which was filtered to remove cell debris, and the clarified solution was collected. The target protein was captured using a kappa select chromatography column. After filtration, the target protein was determined for the protein concentration by dividing the A280 absorbance value measured with a Nanodrop by the extinction coefficient. The final yield was calculated by multiplying the concentration by the volume. The protein was subjected to gel electrophoresis (SDS-PAGE), size exclusion chromatography (SEC-HPLC), and endotoxin detection, confirming the final product as 25G7-Fab.

## Example 4: Preparation process of AZD1402-TAG protein

AZD1402-TAG (the protein sequence was derived from SEQ ID NO 1 in patent WO 2020200960 A1. For the convenience of protein preparation and purification, a His tag was fused to the C-terminus of the sequence, and the final product was named AZD1402-TAG).
AZD1402-TAG

ASDEEIQDVSGTWYLKAMTVDSRCPRAVYNSVTPMTLTTLEGGNLEA
KFTAQRKGRWQKYKLVLEKTDEPGKYTASGGRHVAYIIRSHVKDHYIFHS
EGLCPGQPVPGVWLVGRDPKNNLEALEDFEKAAGARGLSTESILIPRQSET
SSPGSDHHHHHH

SEQ ID NO: 13

The gene sequence encoding the above protein was synthesized and subcloned into pcDNA3.4. After mixing the expression plasmid with the transfection reagent, the mixture was incubated at 37°C for 15 minutes. The mixture was added dropwise to the HEK293 cell suspension. The cell suspension was cultured on a shaker at 37°C for one week. Subsequently, the cell culture was centrifuged and the supernatant was collected. The nickel affinity chromatography column was equilibrated with an imidazole-free buffer, then the protein sample was loaded by passing it through the nickel affinity chromatography column. After re-equilibrating with the imidazole-free buffer again, the column-bound protein was eluted with a buffer containing high-concentration imidazole. The eluted protein was transferred into a dialysis bag and dialyzed against 1×PBS to replace the initial buffer with PBS storage buffer. The target protein was obtained as confirmed by detection.

**Example 5: Preparation of antibody-drug conjugate ADC-1**

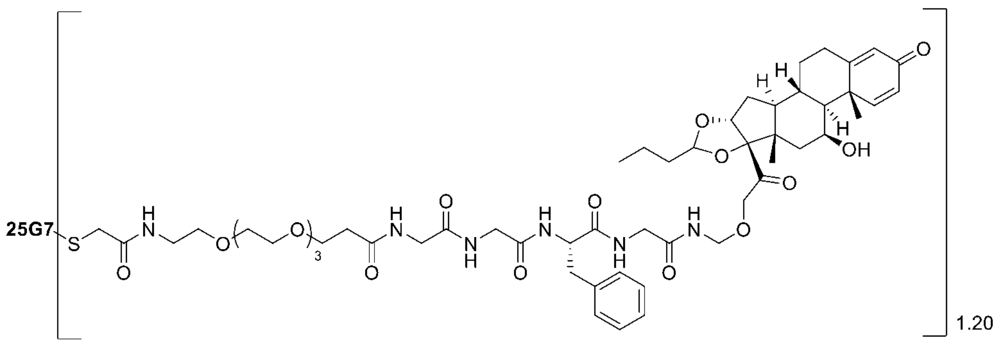

ADC-1

Formulation of Buffer A: In a 2.0 L container, $KH_2PO_4$ (8.50 g), $K_2HPO_4$ (8.56 g), NaCl (5.86 g), and EDTA (1.50 g) were added. Then 1.6 L of water for injection was added, and stirred for half an hour to allow the mixture to be dissolved completely, then water for injection was added to make up the volume to 2.0 L, and the pH was measured as 6.30 ± 0.1.

At 37°C, to an aqueous solution of antibody 25G7-Fab (with the heavy chain sequence as set forth in SEQ ID NO: 11, and the light chain sequence as set forth in SEQ ID NO: 10) in buffer A (0.05 M aqueous buffer solution at pH=6.3; 10.0 mg/ml, 0.5 mL, 0.125 mmol), the formulated aqueous solution of tris(2-carboxyethyl)phosphine (TCEP) (2.5 mM, 100.0 uL, 0.250 mmol) was added. The mixture was placed on a water bath shaker, oscillated for reaction at 37°C for 3 hours, and the reaction was terminated. The reaction solution was cooled to 25°C in a water bath.

1.0 M Tris buffer (50 uL) was added to the above reaction solution, then compound **1** (1.43 mg, 1.250 mmol) was dissolved in 25 ul DMSO, and added to the above reaction solution. The mixture was placed on a water bath shaker, and oscillated for reaction at 25°C for 3 hours, and the reaction was terminated. The reaction solution was desalted and purified using a Sephadex G25 gel column (elution phase: buffer A) and concentrated using an ultrafiltration tube to obtain the title product ADC-1 in buffer A (6.06 mg/mL, 0.60 mL), which was stored at 4°C under frozen conditions.

**Example 6: Preparation of antibody-drug conjugate ADC-2**

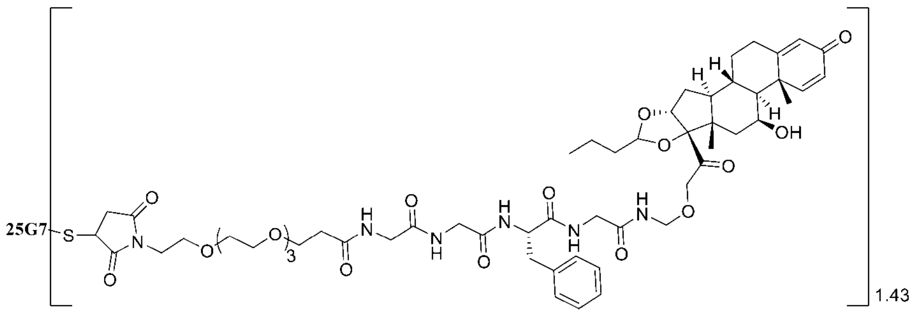

ADC-2

At 37°C, to an aqueous solution of antibody 25G7-Fab in buffer A (0.05 M aqueous buffer solution at pH = 6.3; 10.0 mg/ml, 6.0 mL, 1.50 mmol), the formulated aqueous solution of tris(2-carboxyethyl)phosphine (TCEP) (10.0 mM, 1.50 mL, 15.0 mmol) was added. The mixture was placed on a water bath shaker, and oscillated for reaction at 37°C for 3 hours, and the reaction was terminated. The reaction solution was cooled to 25°C in a water bath.

Compound **2** (12.2 mg, 12.0 mmol) was dissolved in 300 ul DMSO, and added to the above reaction solution. The mixture was placed on a water bath shaker, and oscillated for reaction at 25°C for 3 hours, and the reaction was terminated. The reaction solution was desalted and purified using a Sephadex G25 gel column (elution phase: buffer A) and concentrated using an ultrafiltration tube to obtain the title product ADC-2 in buffer A (3.65 mg/mL, 12.8 mL), which was stored at 4°C under frozen conditions.

**Biological evaluation**

The present disclosure will be further described and explained below in conjunction with test examples, but these examples are not intended to limit the scope of the present disclosure.

**Test example 1: Detection of affinity of antibody-drug conjugate ADC-2 for human-derived IL-4Rα protein**

1. Test proteins

Antibody-drug conjugate ADC-2, 25G7-Fab, and AZD1402-TAG

2. Experimental method

Surface plasmon resonance (SPR) was used to detect the affinity of antibody-drug conjugate ADC-2, 25G7-Fab and AZD1402-TAG for human-derived IL-4Rα antigen protein. An SA sensor chip (Cytiva) was placed in Biacore 8K (Cytiva). Biotin-labeled human-derived IL-4Rα protein (SinoBiological, CAT# 10402-H08H-B) was captured on the SA sensor chip, and HBS-EP+ buffer (10 mM HEPES, 150 mM NaCl, 3 mM EDTA, 0.05% surfactant P20) was used as the mobile phase. Each test protein was serially diluted 2-fold with HBS-EP+ buffer and used as the analyte for injection. The flow rate was set at 30 μL/min, and the detection times for ADC-2, 25G7-Fab, and AZD1402-TAG were 100 seconds for association and 600 seconds for dissociation. Analysis was performed using Biacore 8K evaluation software (Cytiva) to obtain the affinity data of each test protein.

3. Experimental results

Table 1. Affinity KD values for binding of test proteins to human-derived IL-4Rα protein

| Capture protein | Test proteins | Kon (1/Ms) | Koff (1/s) | KD (pM) |
|---|---|---|---|---|
| hIL-4Rα | ADC-2 | 8.78E+06 | 9.62E-05 | 11.0 |
| | 25G7-Fab | 4.69E+06 | 2.06E-04 | 43.9 |
| | AZD1402-TAG | 4.77E+06 | 1.02E-04 | 21.3 |

The results are as shown in Table 1, ADC-2 binds to human-derived IL-4Rα antigen protein with a KD value of 11.0 pM, slightly better than AZD1402-TAG and 25G7-Fab.

**Test example 2: Whole-cell binding activity of antibody-drug conjugate ADC-2 to human-derived IL-4Rα expressing cells**

1. Test proteins

Antibody-drug conjugate ADC-2, 25G7-Fab, hu25G7 (with the heavy chain sequence as set forth in SEQ ID NO: 9, and the light chain sequence as set forth in SEQ ID NO: 10), and AZD1402-TAG.

2. Experimental method

TF-1 and Karpas 299 cell lines were identified to express IL-4Rα. TF-1 and Karpas299 cells in the logarithmic growth phase were collected, counted, adjusted for cell density, and seeded into a round-bottom 96-well plate at $1 \times 10^5$ cells per well. Cells were incubated with various concentrations of anti-IL-4R antibody (25G7, naked anti-25G7-Fab), antibody-drug conjugate ADC-2 and AZD1402-TAG at 4°C for 45 minutes. After washing away antibodies with FACS Buffer (PBS with 2% FBS), fluorescently labeled secondary antibody was added for staining. FITC-conjugated anti-human IgG (Fab-specific) secondary antibody (Sigma, CAT#F5512-1ML) was used for 25G7-Fab, hu25G7 and ADC-2, and FITC-conjugated anti-His antibody (GenScript, CAT#A01620) was used for AZD1402-TAG. The cells were incubated with secondary antibodies at 4°C for 30 minutes, followed by washing with FACS Buffer twice, and detecting by flow cytometer (BD, FACS Celesta). The detection results were analyzed using FlowJo (FlowJo, LLC) software.

3. Experimental results

Table 2. $EC_{50}$ values of whole cell binding activity of test proteins to human-derived IL-4Rα expressing cells

| Test proteins | ADC-2 | 25G7-Fab | hu25G7 | AZD1402-TAG |
|---|---|---|---|---|
| Detection secondary antibodies | Anti-hFab-FITC | | | Anti-His-FITC |
| $EC_{50}$ (nM, TF-1) | 0.107 | 0.243 | 0.247 | 0.0124 |

As shown in FIG. 1 and Table 2, the $EC_{50}$ value of the binding of ADC-2 to TF-1 cells was 0.107 nM, which was comparable to that of the naked antibody 25G7-Fab and hu25G7. AZD1402-TAG exhibited strong binding to TF-1 cells; however, due to the use of different detection antibodies, the data was not comparable to that of ADC-2, 25G7-Fab, and the like.

**Test example 3: Blocking effect of the antibody-drug conjugate ADC-2 on the IL-4/IL-13 signaling pathway**

1. Test proteins

Antibody-drug conjugate ADC-2, and 25G7-Fab.

2. Experimental method

Activation of STAT6 is a key step in the activation of the IL-4/IL-13 signaling pathway. In this example, the blocking effect of ADC-2 on the IL-4/IL-13 signaling pathway was assessed by the HEK-Blue™ IL-4/IL-13 reporter cell line. The cell was purchased from Invivogen (Cat# hkb-i1413), which overexpresses the human-derived STAT6 gene and a secreted alkaline phosphatase (SEAP) reporter gene that is inducibly expressed by phosphorylated STAT6. The activation level of the IL-4/IL-13 signaling pathway can be evaluated by detecting the content of secreted SEAP in the cell culture supernatant using the SEAP substrate QUANTI-Blue.

HEK-Blue™ IL-4/IL-13 cells in the logarithmic growth phase were collected and adjusted to a density of $5 \times 10^5$ cells/ml. A total of 100 μL of the cell suspension was added to each well of a 96-well flat-bottom culture plate, followed by incubation at 37°C and 5% $CO_2$ for 24 hours. Subsequently, 20 μL of serially diluted test proteins and 20 μL of recombinant human IL-4 or IL-13 were added to each well, and the cell culture plate was further incubated in an incubator at 37°C and 5% $CO_2$ for 20-24 hours. After completion of incubation, the cell culture plate was removed. 20 μL of cell culture supernatant was pipetted from each well to another 96-well plate, and 180 μL of pre-warmed (37°C) QUANTI-Blue chromogenic solution was added to each well. Following incubation at 37°C for 1 hour, the absorbance value was measured at a wavelength of 620 nm.

3. Experimental results

Table 3. $IC_{50}$ values of test proteins for blocking the IL-4/IL-13 signaling pathway

| Test proteins | ADC-2 | 25G7-Fab |
|---|---|---|
| $IC_{50}$ values for blocking the IL-4 signaling pathway (nM) | 0.68 | 0.61 |
| $IC_{50}$ values for blocking the IL-13 signaling pathway (nM) | 10.60 | 14.59 |

The results are as shown in FIG.2 and Table 3, all test proteins exhibited blocking effects on the activation of STAT6 induced by recombinant human IL-4 and IL-13. The $IC_{50}$ value of ADC-2 for blocking the IL-4 signaling pathway was 0.68 nM, which was close to the $IC_{50}$ value of 25G7-Fab, 0.61 nM. The $IC_{50}$ value of ADC-2 for blocking the IL-13 signaling pathway was 10.60 nM, which was comparable to the $IC_{50}$ value of 25G7-Fab, 14.59 nM.

**Test example 4: Endocytosis activity for antibody-drug conjugate ADC-2 in TF-1 cells**

1. Test proteins

Antibody-drug conjugate ADC-2, 25G7-Fab, hu25G7, and AZD1402-TAG.

2. Experimental method

After collecting TF-1 cells in good growth condition, the density was adjusted to $1 \times 10^6$ cells/mL. 100 μL of the cell suspension was added to each well of a 96-well cell culture plate and the plate was placed at 4°C. Each test protein at a final concentration of 2 nM was added, incubated at 4°C for 1 hour, then placed into a cell incubator and cultured continuously at 4°C and 5% $CO_2$. A single separate culture plate was set for each time point. During the culture, the culture plate was removed at different incubation time points, and washed with FACS buffer followed by centrifugation, and then a fluorescently labeled secondary antibody was added for incubation. 25G7-Fab, hu25G7, and ADC-2 were labeled with a FITC-conjugated anti-human IgG (Fab-specific) secondary antibody, while AZD1402-TAG was labeled with a FITC-conjugated anti-His antibody. The cells were incubated with secondary antibodies at 4°C for 30 minutes, followed by washing with FACS Buffer twice, and detecting by flow cytometer (BD, FACS Celesta). The detection results were analyzed using FlowJo (FlowJo, LLC) software. The formula for calculating the endocytosis rate at a specific time point is:

$$(gMFI_{\text{Test Ab at time X}} - gMFI_{\text{ISO Ab at time X}})/(gMFI_{\text{Test Ab at time zero}} - gMFI_{\text{ISO Ab at time zero}})*100\%$$

3. Experimental results

The experimental results are as shown in FIG. 3, and endocytosis signals of all test proteins are detectable in TF-1 cells. The endocytosis activity for ADC-2 and hu25G7 was comparable, with endocytosis rates around 70% at 3 hours; and for 25G7-Fab, the endocytosis effect was faster and the endocytosis rate was also higher. Compared with AZD1402-TAG, the endocytosis speeds and endocytosis rates for ADC-2, 25G7-Fab and hu25G7 were significantly superior to AZD1402-TAG, the plateau was reached at about 2 hours, and the endocytosis activity for ADC-2 was slightly weaker than 25G7-Fab.

**Test example 5: Pharmacokinetic test of the antibody-drug conjugate ADC-2 in a mouse model**

1. Test proteins

Antibody-drug conjugate ADC-2

2. Experimental method

2.1 Experimental animals

C57BL6/J mice, 8 weeks old, male, Cyagen (Suzhou) Biosciences Inc.

2.2 Drug administration in mice

Mice received intratracheal (i.t.) nebulized administration. At different time points after administration, 300-400 μL of blood was collected from the mandible. After standing for 2 hours, the blood samples were centrifuged at 7500 rpm and 4°C for 10 min, and the serum was harvested. Following blood collection, the mice were anesthetized and immobilized. The cervical muscles of the mice were bluntly dissected to expose the trachea, which was then transected horizontally. A lavage needle was inserted slowly, and pre-cooled normal saline (4°C) that had been drawn up in advance was slowly injected into the lung tissue for lavage, followed by collection of bronchoalveolar lavage fluid (BALF). A total of 3 lavages were performed: 300 μL of normal saline was used for each of the first two lavages, and 400 μL for the third. The collected bronchoalveolar lavage fluid was centrifuged at 1000 rpm and 4°C for 10 min, and the supernatant was collected. After euthanizing the mice, their thoracic cavities were opened, and the intact mouse lung tissues were dissected and collected. The lung tissues were rinsed with PBS to remove surface blood stains, and after blotting the surface PBS dry with filter paper, the lung tissues were weighed. The lung tissues were placed into 2 mL centrifuge tubes, 1 mL of cell lysis buffer (CST, #9803) was added, and the tissues were ground at 60 Hz for 2 min using a homogenizer (Shanghai Jing Xin, Tissuelyser-48). After centrifugation at 10,000 × g and 4°C for 10 min, the supernatant was the lung tissue homogenate, and the protein concentration of the homogenate was determined (Pierce, 23227). The serum, BALF, and lung tissue homogenate were stored at -80°C. The administration scheme is shown in Table 4, and the sampling scheme is shown in Table 5.

Table 4. Administration scheme

| Group | Number of animals (n) | Test proteins | Dose (μg) | Route of administration | Frequency of administration |
|---|---|---|---|---|---|
| ADC-2 group | 15 | ADC-2 | 100 | i.t. | Once |

Table 5. Sampling scheme

| Time after administration | | | | | | |
|---|---|---|---|---|---|---|
| 5 min | 1 h | 6 h | 24 h | 48 h | Pre-dose | 30 min |
| Animal number | | | | | | |
| 1,2,3 | 4, 5, 6 | 7, 8, 9 | 10, 11, 12 | 13, 14, 15 | 10, 11, 12 | 13, 14, 15 |
| At this point, serum was first collected, animals were sacrificed, and BALF and lung tissue were then collected. | | | | | At this time point, only serum was collected. | |

2.3 The content detection of ADC-2

(1) Coating: The antigen hIL-4Rα (Acro, ILR-H5221) was diluted to 1 μg/mL with carbonate buffer, and 100 μL of the resulting solution was added to each well of the ELISA plate, and incubated overnight (16-18 h) at 4°C.
(2) Washing the plate: The ELISA plate was brought to room temperature and washed 3 times with 300 μL of 0.05% PBST (PBS + 0.05% Tween20) buffer per well.
(3) Blocking: 300 μL PBS with 3% BSA was added to each well, and the plate was incubated at room temperature under shaking at 400 rpm for 2 h.
(4) Washing the plate: 300 μL of 0.05% PBST was added to each well and the plate was washed 3 times.
(5) Sample incubation: 100 μL of diluted serum, BALF or lung tissue homogenate was added to the ELISA plate, and the plate was incubated at room temperature under shaking at 400 rpm for 1.5 h.
(6) Washing the plate: 300 μL of 0.05% PBST was added to each well and the plate was washed 3 times.
(7) Detection: Detection antibody (Invitrogen, A18811) was diluted 10000-fold with an assay buffer (0.05% PBST with 0.5% BSA), 100 μL of the resulting solution was added to each well of the ELISA plate, and the plate was incubated at room temperature under shaking at 400 rpm for 1 h.
(8) Washing the plate: 300 μL of 0.05% PBST was added to each well and the plate was washed 3 times.

(9) Color development: 100 $\mu$L. TMB substrate (Sera care, 5120-0080) was added to each well and incubated for 15 min at room temperature.

(10) Termination: 100 $\mu$L of substrate reaction stop solution (Solarbio, C1058) was added to each well and the absorbance value at 620 nm was measured.

2.4 The content detection of free budesonide

30 $\mu$L of serum, BALF, or lung tissue homogenate were taken, 30.0 $\mu$L of internal standard working solution (5.00 ng/mL budesonide-D8) and 120 $\mu$L of acetonitrile were added, the mixture was vortexed for 1 min, centrifuged at 13,000 rpm and 4°C for 5 min. 100 $\mu$L of the supernatant were added to a 96-well plate, 100 $\mu$L of 0.3% FA diluent was added, the plate was incubated at room temperature under shaking at 1,000 rpm for 15 min, and 20 $\mu$L of the resulting solution was taken for LC-MS/MS analysis (Shimadzu Corporation, Japan; Shimadzu liquid chromatography system; AB Sciex, Triple Quad 6500$^+$ triple quadrupole tandem mass spectrometer).

3. Experimental results

The pharmacokinetic parameters of the antibody-drug conjugate ADC-2 and free budesonide are shown in Table 6, and the drug concentration-time curve is shown in FIG. 4.

Table 6. PK parameters of ADC-2 and free budesonide

| | **ADC-2** | | | **Free budesonide** | | |
|---|---|---|---|---|---|---|
| **Pharmacokinetic parameter** | **Serum** | **BALF** | **Lung tissue** | **Serum** | **BALF** | **Lung tissue** |
| Peak drug concentration ($C_{max}$, ng/mL) | 2427 | 31377 | 7293 | 0.7 | 1.1 | 1.88 |
| Time to peak drug concentration ($T_{max}$, h) | 1.0 | 1.0 | 1.0 | 1.0 | 0.083 | 6.0 |
| Area under the drug concentration-time curve ($AUC_{last}$, ng/mL·h) | 28526 | 451418 | 133942 | 3.43 | 0.046 | 53.72 |
| Half-life ($T_{1/2}$, h) | 9.4 | 13.8 | 10.7 | / | / | / |
| Clearance (CL, g/h) | 3.41 | 0.20 | 0.72 | / | / | / |
| Apparent volume of distribution (Vz, g) | 46.46 | 4.06 | 11.17 | / | / | / |

Experimental results show that after intratracheal administration of the antibody-drug conjugate ADC-2, the drug is mainly distributed in serum and BALF, with low serum exposure. Exposure of budesonide was very low in all tissues.

**Test example 6:** In vivo efficacy of ADC-2 in an OVA-induced asthma model in mice

1. Test

Antibody-drug conjugate ADC-2, 25G7-Fab, AZD1402-TAG, and budesonide.

2. Experimental method

2.1 Experimental animals

IL-4 hu/hu-IL-4R$\alpha$ hu/hu double transgenic C57BL6/J mice, 8 weeks old, female, Cyagen (Suzhou) Biosciences Inc.

2.2 Drug administration in mice

Animals were randomized into normal control and modeling animals. On days 0, 7, and 14, the mice were sensitized by intraperitoneal injection of 100 $\mu$L of a PBS solution containing 200 $\mu$g OVA (Sigma, A5503) with an equal volume of aluminum hydroxide as an adjuvant. On days 21-27, mice were allowed to inhale 3% OVA nebulized solution once a day, with each session lasting 35 min. Beginning on day 21, the mice received intratracheal nebulized administration half an hour before inhalation of OVA nebulized solution. The specific administration scheme is shown in Table 7.

On day 28, after euthanizing the mice, the cervical muscles of the mice were bluntly dissected to expose the trachea, which was then transected horizontally. A lavage needle was inserted slowly, and pre-cooled normal saline (4°C) that had been drawn up in advance was slowly injected into the lung tissue for lavage, followed by collection of bronchoalveolar lavage fluid. A total of 3 lavages were performed: 300 μL of normal saline was used for each of the first two lavages, and 400 μL for the third. The fluid was centrifuged at 1,000 rpm and 4°C for 10 min, the cell pellet was resuspended in 350 μL of pre-cooled normal saline, and the leukocytes and subpopulations were counted using a fully automated hematology analyzer.

Table 7. Administration scheme

| **Group** | **Number of animals (n)** | **Test proteins** | **Administrati on dose (μg)** | **Route of administrati on** | **Frequency of administrati on** |
|---|---|---|---|---|---|
| Normal control group | 6 | PBS | / | i.t. | QD |
| Modeling group | 6 | PBS | / | i.t. | QD |
| ADC-2 group | 8 | ADC-2 | 100 | i.t. | QD |
| AZD1402-TAG group | 8 | AZD 1402 -TAG | 30 | i.t. | QD |
| 25G7-Fab group | 8 | 25G7-Fab | 100 | i.t. | QD |
| Budesonide group | 6 | Budesoni de | 1 | i.t. | QD |

2.3 Experimental indicators

The number of leukocytes and subpopulations in BALF.

2.4 Statistical analysis

Unless otherwise specified, comparisons of cell counts between two groups were performed using one-way ANOVA test, with $p < 0.05$ defined as statistically significant.

3. Experimental results

The experimental results are as shown in FIG. 5. Compared with the modeling group, at an equivalent molar dose, the antibody-drug conjugate ADC-2 significantly reduces the number of leukocytes, eosinophils, and neutrophils in BALF, and also moderately suppresses the number of monocytes. It demonstrates superior efficacy to AZD1402-TAG and is markedly more effective than both 25G7-Fab and budesonide. ADC-2 demonstrates a synergistic effect combining 25G7-Fab and budesonide in efficacy.

**Test example 7:** The dose exploration for in vivo efficacy of ADC-2 in an OVA-induced asthma model in mice

1. Test

Antibody-drug conjugate ADC-2, AZD1402-TAG, and budesonide.

2. Experimental method

2.1 Experimental animals

IL-4 hu/hu-IL-4Rα hu/hu double transgenic C57BL6/J mice, 8 weeks old, female, Cyagen (Suzhou) Biosciences Inc.

2.2 Drug administration in mice

Administration and BALF sampling were performed as in Test example 6. The specific administration scheme is shown in Table 8.

Table 8. Administration scheme

| Group | Number of animals (n) | Test proteins | Administrat ion dose ($\mu g$) | Route of administrat ion | Frequency of administration |
|---|---|---|---|---|---|
| Normal control group | 8 | PBS | / | i.t. | QD |
| Modeling group | 8 | PBS | / | i.t. | QD |
| ADC-2 low dose group | 8 | ADC-2 | 10 | i.t. | QD |
| ADC-2 medium dose group | 8 | ADC-2 | 30 | i.t. | QD |
| ADC-2 high dose group | 8 | ADC-2 | 100 | i.t. | QD |
| AZD1402-TAG group | 8 | AZD 140 2-TAG | 30 | i.t. | QD |
| Budesonide group | 6 | Budesoni de | 1 mpk | i.t. | QD |

2.3 Experimental indicators

The number of leukocytes and subpopulations in BALF.

2.4 Statistical analysis

Unless otherwise specified, comparisons of cell counts between two groups were performed using one-way ANOVA test, with $p < 0.05$ defined as statistically significant.

3. Experimental results

The experimental results are as shown in FIG. 6. Compared with the modeling group, 10 $\mu g$ of ADC-2 significantly reduces the number of eosinophils in bronchoalveolar lavage fluid, which is comparable to AZD1402-TAG, and slightly better than budesonide at a dose which is much higher than the clinical equivalent dose used. In addition, 10 $\mu g$ of ADC-2 also has a significant inhibitory effect on the number of leukocytes, with some extent of inhibition of the number of monocytes and neutrophils. These results indicate that 1/10 molar dose of ADC-2 can achieve comparable efficacy to AZD1402-TAG.

**Example 7: Preparation of dry powders a-d for inhalation**

Table 9. Compositions of formulations a-d

| No. | a | b | c | d |
|---|---|---|---|---|
| ADC-2 | 2 mg/ml | 2 mg/ml | 2 mg/ml | 2 mg/ml |
| Sodium sulfate | 0.426 mg/ml | 0.426 mg/ml | N/A | N/A |
| Sodium citrate | N/A | N/A | 0.426 mg/ml | 0.426 mg/ml |
| Trehalose | 0.3 mg/ml | 0.3 mg/ml | 0.3 mg/ml | 0.3 mg/ml |
| Histidine | 0.1824 mg/ml | 0.3 mg/ml | 0.3 mg/ml | 0.3 mg/ml |
| Histidine hydrochloride | 0.1176 mg/ml | N/A | N/A | N/A |
| Isopropanol | 5% v/v | 5% v/v | 5% v/v | 5% v/v |
| Purified water | 95% v/v | 95% v/v | 95% v/v | 95% v/v |
| pH | 5.0 | 5.0 | 5.0 | 5.5 |

Preparation process:

1) The solution of auxiliary materials was prepared by adding water to the formulation composition in Table 9, then a prescribed amount of ADC-2 buffer (prepared in Example 6) was added, and the mixture was stirred gently until homogeneous. A prescribed amount of isopropanol was added. The mixture was stirred gently until homogeneous (pH can be adjusted with hydrochloric acid if necessary).
2) Freeze-drying.

The particle size measurement was carried out using a SYMPATEC particle size measuring instrument with the dispersion pressure set at 4 bar, and the particle size results are shown in Table 10.

Table 10. Geometric particle size determination results of dry powders a-c for inhalation

| No. | X10 (μm) | X50 (μm) | X90 (μm) |
|---|---|---|---|
| a | 0.50 | 1.42 | 3.14 |
| b | 0.56 | 1.40 | 2.95 |
| c | 0.53 | 1.46 | 3.75 |

The prepared drug-containing dry powder for inhalation was filled into transparent HPMC capsules (size 3) with a filling weight of 10 mg. The capsule samples were tested for APSD using an NGI impactor (COPLEY Scientific Ltd.) in accordance with the requirements specified in General Chapter 0951 [Determination of Aerodynamic Properties of Fine Particles in Inhalation Preparations] of the 2015 Edition of the Chinese Pharmacopoeia (Volume IV). The distribution results of each stage of the APSD were input into CITDAS version 3.10 software (COPLEY Scientific Ltd.) to obtain values such as FPF (< 5 μm), MMAD, and GSD. The results are shown in Table 11.

Table 11. Aerodynamic particle size (APSD) test results of dry powders for inhalation

| 60 L/min | a | b | c | d |
|---|---|---|---|---|
| $FPD_{<5\mu m}$ (mg) | 2.708 | 2.533 | 1.834 | 2.285 |
| ED (mg) | 4.37 | 4.21 | 4.80 | 4.03 |
| FPF (<5 μm), % (over ED) | 62.0% | 60.1% | 38.2% | 56.7% |
| MMAD (μm) | 3.005 | 3.012 | 3.691 | 3.266 |
| GSD | 2.012 | 1.995 | 2.175 | 1.869 |
| 30 L/min | a | b | c | d |
| $FPD_{<5\mu m}$ (mg) | 1.382 | 1.116 | 1.119 | 1.500 |
| ED (mg) | 3.67 | 3.32 | 5.09 | 3.96 |
| FPF (<5 μm), % (over ED) | 37.7% | 33.7% | 22.0% | 37.8% |
| MMAD (μm) | 4.709 | 4.972 | 5.863 | 4.619 |
| GSD | 2.028 | 1.985 | 1.966 | 1.976 |

The samples were placed under different stability test conditions, and analyzed for the drug stability using the SEC method (size exclusion chromatography). The results are shown in Table 12. The results indicate that formulations c and d exhibited the smallest decrease in monomer content during the stability study, and the addition of sodium citrate to the formulations enhanced the stability of the macromolecular properties.

Table 12. Purity determination (SEC) results (%) of active ingredients

| No. | Stability condition | Aggregates | Naked antibodies | Monomers | Fragments | Unknown impurities |
|---|---|---|---|---|---|---|
| a | 0 days | 0.83 | 1.90 | 96.6 | 0.68 | N/A |
| | 40°C, 35 days | 3.78 | 1.89 | 87.24 | 1.04 | 6.05 |
| | 25°C, 35 days | 1.59 | 2.00 | 95.71 | 0.70 | N/A |
| | 2-8°C, 35 days | 1.14 | 1.78 | 96.25 | 0.83 | N/A |
| b | 0 days | 0.72 | 1.8 | 96.8 | 0.61 | N/A |
| | 40°C, 35 days | 3.43 | 2.18 | 86.81 | 1.19 | 6.39 |
| | 25°C, 35 days | 1.39 | 1.99 | 95.44 | 1.17 | N/A |
| | 2-8°C, 35 days | 1.01 | 1.87 | 95.96 | 1.15 | N/A |
| c | 0 days | 0.52 | 1.80 | 97.2 | 0.49 | N/A |
| | 40°C, 35 days | 2.25 | 2.77 | 91.42 | 0.81 | 2.75 |
| | 25°C, 35 days | 0.68 | 2.41 | 96.14 | 0.76 | N/A |
| | 2-8°C, 35 days | 0.60 | 2.01 | 96.86 | 0.53 | N/A |
| d | 0 days | 0.49 | 1.80 | 97.2 | 0.49 | N/A |
| | 40°C, 35 days | 3.14 | 1.95 | 90.67 | 0.97 | 3.28 |
| | 25°C, 35 days | 1.18 | 1.92 | 96.25 | 0.65 | N/A |
| | 2-8°C, 35 days | 0.85 | 1.79 | 96.66 | 0.70 | N/A |

**Example 8: Preparation of dry powders e, f, and g for inhalation**

The same formulation composition and preparation method was used as for formulation b of Example 7, except that the amount of the organic solvent used was adjusted, as shown in Table 13.

Table 13. The amount of water and isopropanol used in the preparation of dry powders e, f, and g for inhalation

| No. | Isopropanol v/v | Water v/v |
|---|---|---|
| e | 2% | 98% |
| f | 5% | 95% |
| g | 10% | 90% |

The particle size measurement was carried out using a SYMPATEC particle size measuring instrument with the dispersion pressure set at 4 bar, and the particle size results are shown in Table 14.

Table 14. Geometric particle size determination results of dry powders e, f and g for inhalation

| No. | X10 (μm) | X50 (μm) | X90 (μm) |
|---|---|---|---|
| e | 0.48 | 1.59 | 3.66 |
| f | 0.51 | 1.45 | 3.94 |
| g | 0.49 | 1.46 | 4.33 |

**Example 9: Preparation of dry powders h-k for inhalation**

Powders for inhalation were prepared according to the formulation composition in Table 15 using the same formulation and lyophilization process as in Example 7.

Table 15. Compositions of formulations h-k

| No. | h | i | i | k |
|---|---|---|---|---|
| ADC-2 | 2 mg/ml | 2.5 mg/ml | 3 mg/ml | 3.5 mg/ml |
| Sodium sulfate | 0.426 mg/ml | 0.5325 mg/ml | 0.639 mg/ml | 0.7455 mg/ml |
| Trehalose | 0.3 mg/ml | 0.375 mg/ml | 0.45 mg/ml | 0.525 mg/ml |
| Histidine | 0.3 mg/ml | 0.375 mg/ml | 0.45 mg/ml | 0.525 mg/ml |
| Isopropanol | 5% v/v | 5% v/v | 5% v/v | 5% v/v |
| Purified water | 95% v/v | 95% v/v | 95% v/v | 95% v/v |
| pH | 5.5 | 5.5 | 5.5 | 5.5 |

The particle size measurement was carried out using a SYMPATEC particle size measuring instrument with the dispersion pressure set at 4 bar, and the particle size results are shown in Table 16. The results show that when the drug concentration is increased from 2 mg/ml to 3.5 mg/ml, the change in geometric particle size is insignificant.

Table 16. Geometric particle size determination results of dry powders h-k for inhalation.

| No. | X10 (μm) | X50 (μm) | X90 (μm) |
|---|---|---|---|
| h | 0.55 | 1.53 | 3.77 |
| i | 0.49 | 1.78 | 4.22 |
| j | 0.49 | 1.89 | 4.62 |
| k | 0.55 | 1.94 | 4.67 |

The aerodynamic particle size determination method was the same as in Example 7, and the APSD results are shown in Table 17. The results show that the drug concentration changes in the range of 2-3.5 mg/ml have little effect on aerodynamic particle size results such as FPF and MMAD.

Table 17. Aerodynamic particle size (APSD) test results of dry powders for inhalation

| 30 L/min | h | i | j | k |
|---|---|---|---|---|
| $FPD_{<5\mu m}$ (mg) | 1.616 | 1.658 | 1.63 | 1.68 |
| ED (mg) | 4.71 | 4.42 | 4.61 | 5.13 |
| FPF (<5 μm), % (over ED) | 34.3 | 37.5 | 35.4 | 32.8 |
| MMAD (μm) | 4.831 | 4.666 | 4.694 | 4.765 |
| GSD | 2.118 | 2.072 | 2.094 | 2.066 |

**Example 10: Morphologic observation of dry powders for inhalation**

The morphologies of the dry powders c and f were observed using a scanning electron microscope (SEM). The results are shown in FIGS. 7 and 8. It can be seen that both the dry powders c and f for inhalation are composed of aggregates of several nanoscale smooth spheres.

**Example 11: Preparation of dry powders for inhalation of different specifications**

Preparation of dry powder m for inhalation (0.4 mg format): The prescribed amounts of histidine and sodium citrate in Table 18, and 0.06 mg of trehalose were weighed, and water was added to formulate a solution of auxiliary materials. The prescribed amount of ADC-2 buffer (prepared in Example 6) was added. The mixture was stirred gently until homogeneous. The prescribed amount of isopropanol was added. The mixture was stirred gently until homogeneous. The lyophilized powder was prepared using the same lyophilization process as in Example 7, and the remaining prescribed amount of trehalose was added as a filler to fill the hypromellose hollow capsules.

Preparation of dry powder n for inhalation (2 mg format): Powder m for inhalation was prepared according to the

formulation composition in Table 18 using the same formulation and lyophilization process as in Example 7. The powder was directly filled into the hypromellose hollow capsules.

Table 18. Composition of formulations m and n

| No. | m | n |
|---|---|---|
| ADC-2 | 0.4 mg | 2 mg |
| Trehalose | 7.46 mg | 0.30 mg |
| Histidine | 0.06 mg | 0.30 mg |
| Sodium citrate | 0.085 mg | 0.426 mg |
| Isopropanol | 0.01 ml | 0.05 ml |
| Purified water | 0.19 ml | 0.95 ml |
| pH | 5.5 | 5.5 |

The aerodynamic particle size determination method was the same as in Example 7, and the APSD results are shown in Table 19.

Table 19. Aerodynamic particle size (APSD) test results of dry powders for inhalation

| Dry powder n for inhalation | Low flow rate, 30 L/min | Medium flow rate, 60 L/min | High flow rate, 90 L/min |
|---|---|---|---|
| $FPD_{<5\mu m}$ (mg) | 0.626 | 0.946 | 0.838 |
| ED (mg) | 1.48 | 1.58 | 1.55 |
| FPF (<5 μm), % (over ED) | 42.1% | 59.9% | 54.2% |
| MMAD (μm) | 4.364 | 3.109 | 2.763 |
| GSD | 2.084 | 2.065 | 2.091 |
| Dry powder m for inhalation | Low flow rate, 30 L/min | Medium flow rate, 60 L/min | High flow rate, 90 L/min |
| $FPD_{<5\mu m}$ (mg) | 0.066 | 0.083 | 0.093 |
| ED (mg) | 0.26 | 0.29 | 0.29 |
| FPF (<5 μm), % (over ED) | 25.6% | 28.1% | 32.6% |
| MMAD (μm) | 5.773 | 4.845 | 4.351 |
| GSD | 1.838 | 1.977 | 2.047 |

## Claims

**1.** A pharmaceutical composition, comprising an antibody-drug conjugate and a counterion, wherein the antibody-drug conjugate has a structure as shown in formula I:

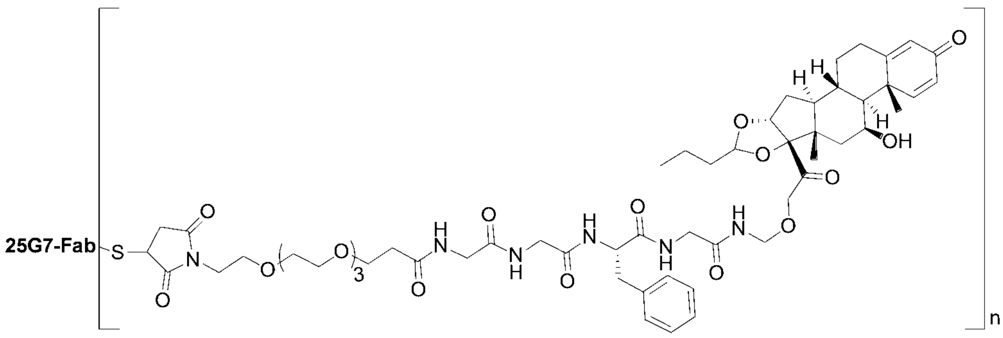

I ;

wherein:

25G7-Fab comprises a heavy chain variable region and a light chain variable region, the heavy chain variable region comprising: HCDR1 as set forth in SEQ ID NO: 1 or SEQ ID NO: 12, HCDR2 as set forth in SEQ ID NO: 2, and HCDR3 as set forth in SEQ ID NO: 3, the light chain variable region comprising: LCDR1 as set forth in SEQ ID NO: 4, LCDR2 as set forth in SEQ ID NO: 5, and LCDR3 as set forth in SEQ ID NO: 6; and
n is 1 to 10.

**2.** The pharmaceutical composition according to claim 1, **characterized in that** the 25G7-Fab has a heavy chain variable region as set forth in SEQ ID NO: 8, and a light chain variable region as set forth in SEQ ID NO: 7.

**3.** The pharmaceutical composition according to claim 1 or 2, **characterized in that** the 25G7-Fab has a heavy chain as set forth in SEQ ID NO: 11 and a light chain as set forth in SEQ ID NO: 10.

**4.** The pharmaceutical composition according to any of claims 1-3, **characterized in that** the concentration of the antibody-drug conjugate is 0.1 mg/mL-50 mg/mL, preferably 0.5 mg/mL-10 mg/mL, more preferably 1 mg/mL-5 mg/mL, based on the protein concentration.

**5.** The pharmaceutical composition according to any of claims 1-4, **characterized in that** the counterion is selected from one or more of sulfate, phosphate, malate, maleate, oxalate, citrate, succinate, fumarate, itaconate, chloride and bromide, preferably one or more of sulfate, citrate and chloride, more preferably one or more of sodium citrate, sodium sulfate, zinc sulfate, magnesium sulfate, potassium sulfate, calcium sulfate, magnesium chloride, calcium chloride and zinc chloride, most preferably sodium sulfate and/or sodium citrate.

**6.** The pharmaceutical composition according to any of claims 1-5, **characterized in that** the concentration of the counterion is 0.01 mg/mL-10 mg/mL, preferably 0.05 mg/mL-5 mg/mL, more preferably 0.1 mg/mL-2 mg/mL.

**7.** The pharmaceutical composition according to any of claims 1-6, **characterized in that** the pharmaceutical composition further comprises a protective agent.

**8.** The pharmaceutical composition according to claim 7, **characterized in that** the protective agent is selected from an amino acid and/or a sugar.

**9.** The pharmaceutical composition according to claim 8, **characterized in that** the amino acid is selected from one or more of glycine, histidine, arginine, lysine, glutamic acid, alanine, valine, leucine, isoleucine, proline, tryptophan, phenylalanine, methionine and aspartic acid, more preferably glycine or histidine.

**10.** The pharmaceutical composition according to claim 8 or 9, **characterized in that** the concentration of the amino acid is 0.01 mg/mL-10 mg/mL, preferably 0.05 mg/mL-5 mg/mL, more preferably 0.1 mg/mL-2 mg/mL.

**11.** The pharmaceutical composition according to claim 8, **characterized in that** the sugar is selected from one or more of sorbitol, mannitol, xylitol, trehalose, lactose, fructose, maltose and sucrose, preferably trehalose.

**12.** The pharmaceutical composition according to claim 11, **characterized in that** the concentration of the sugar is 0.01

mg/mL-10 mg/mL, preferably 0.05 mg/mL-5 mg/mL, more preferably 0.1 mg/mL-2 mg/mL.

**13.** The pharmaceutical composition according to any of claims 1-12, **characterized in that** the pharmaceutical composition further comprises an organic solvent, and the organic solvent is preferably one or several of methanol, ethanol, 1-propanol, isopropanol, tert-butanol, butanol, acetone, acetonitrile, acetic acid, ethyl acetate, methyl ethyl ketone, methyl tert-butyl ether and dimethyl sulfoxide, more preferably isopropanol.

**14.** The pharmaceutical composition according to claim 13, **characterized in that** the amount of the organic solvent is 0.1 %-50% v/v, preferably 0.5%-30% v/v, more preferably 1%-10% v/v.

**15.** The pharmaceutical composition according to any of claims 1-14, **characterized in that** the pharmaceutical composition has a pH of 3.0-8.0, preferably 4.5-6.0.

**16.** The pharmaceutical composition according to any of claims 1-15, **characterized in that** the pharmaceutical composition comprises

a) 1 mg/mL-5 mg/mL of the antibody-drug conjugate based on the protein concentration,
b) 0.1 mg/mL-2 mg/mL of the counterion which is sodium citrate or sodium sulfate,
c) 0.1 mg/mL-2 mg/mL of the amino acid which is histidine or glycine,
d) 0.1 mg/mL-2 mg/mL of the sugar which is trehalose, and
e) 1%-10% v/v of the organic solvent which is isopropanol;

and the pharmaceutical composition has a pH of 4.5-6.0.

**17.** A lyophilized preparation comprising an antibody-drug conjugate, **characterized in that** the lyophilized preparation is obtained by freeze-drying the pharmaceutical composition according to any of claims 1 to 16.

**18.** A reconstituted solution comprising an antibody-drug conjugate, **characterized in that** the reconstituted solution is prepared by reconstituting the lyophilized preparation according to claim 17.

**19.** A pharmaceutical composition, comprising the lyophilized preparation according to claim 17, and a filler, wherein the filler is selected from one or more of trehalose, lactose, mannitol, glucose, sorbitol and dextran, preferably trehalose.

**20.** An article of manufacture, comprising a container containing the pharmaceutical composition according to any of claims 1 to 16, the lyophilized preparation according to claim 17, the reconstituted solution according to claim 18 or the pharmaceutical composition according to claim 19.

**21.** Use of the pharmaceutical composition according to any of claims 1 to 16, the lyophilized preparation according to claim 17, the reconstituted solution according to claim 18, the pharmaceutical composition according to claim 19 or the article of manufacture according to claim 20 in the preparation of a medicament for treating or preventing an immune disease or condition, wherein the disease or condition is preferably: asthma, nasal polyps, chronic sinusitis, allergic skin disease, eosinophilic esophagitis, chronic obstructive pulmonary disease, allergic rhinitis, arthritis, inflammatory diseases, allergic reactions, autoimmune lymphoproliferative syndrome, autoimmune hemolytic anemia, Barrett’s esophagus, autoimmune uveitis, tuberculosis and kidney disease, more preferably asthma or allergic skin disease.

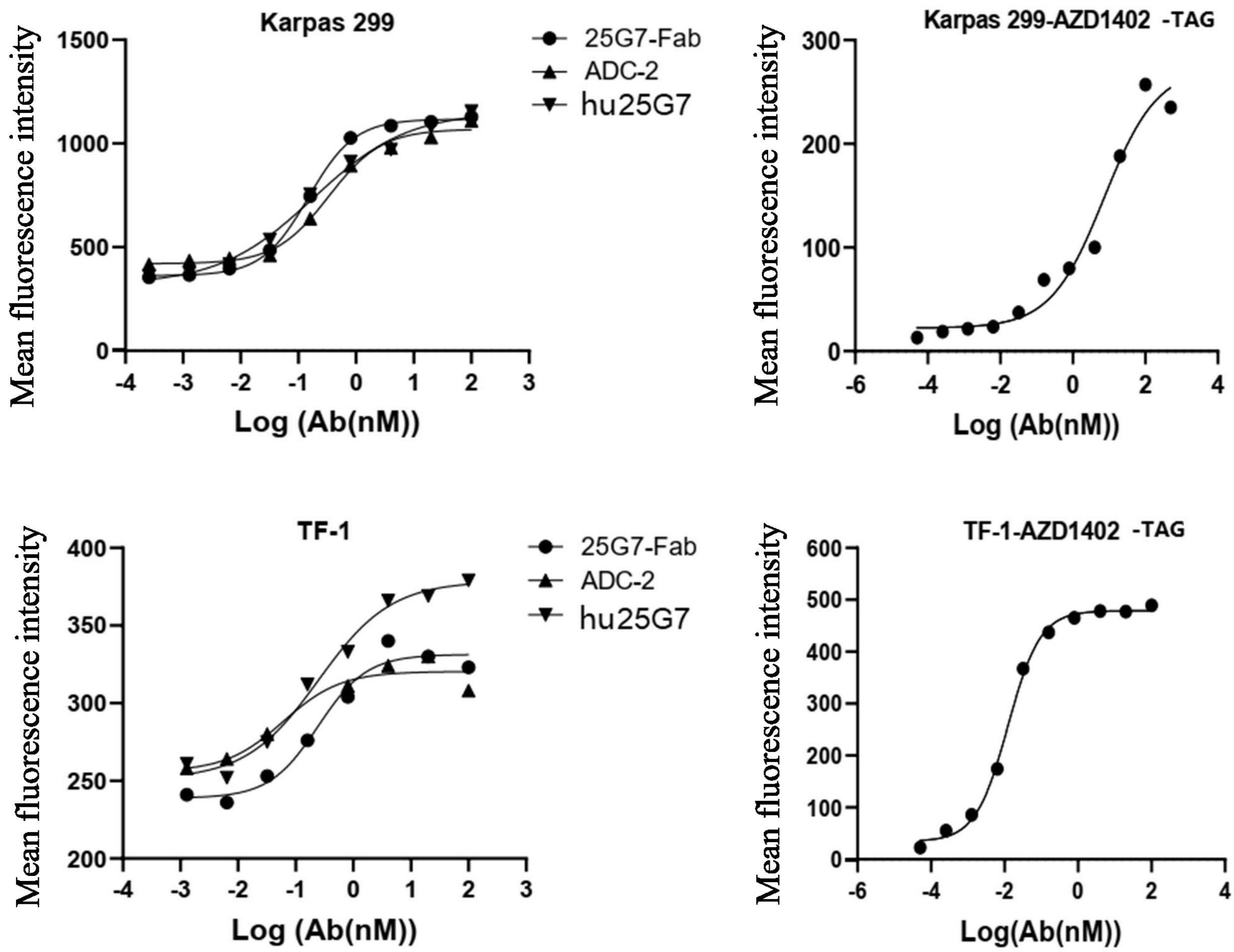
Karpas 299
25G7-Fab
ADC-2
hu25G7
Mean fluorescence intensity
Log (Ab(nM))
Karpas 299-AZD1402 -TAG
Mean fluorescence intensity
Log (Ab(nM))
TF-1
25G7-Fab
ADC-2
hu25G7
Mean fluorescence intensity
Log (Ab(nM))
TF-1-AZD1402 -TAG
Mean fluorescence intensity
Log(Ab(nM))

*FIG. 1*

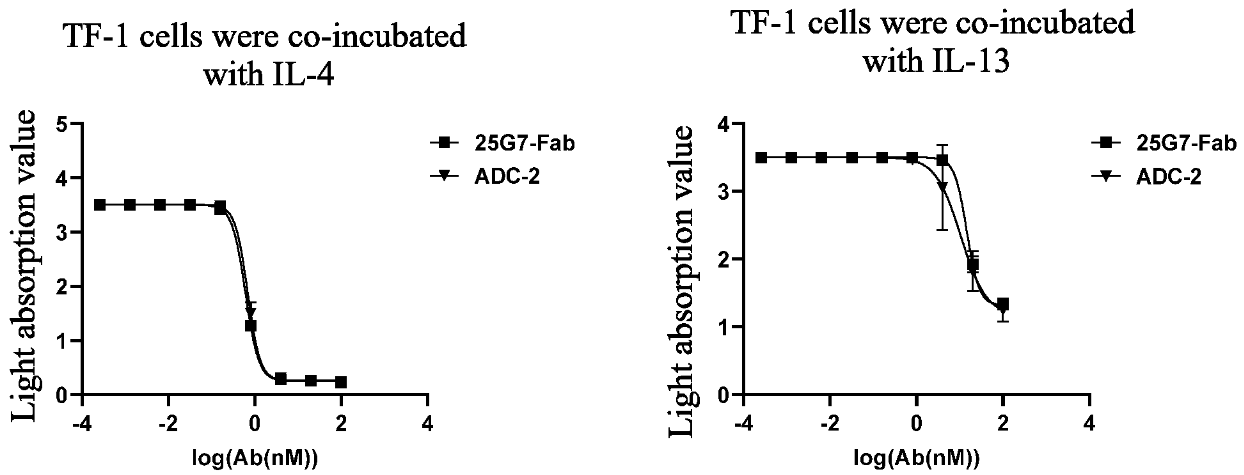
TF-1 cells were co-incubated with IL-4
Light absorption value
log(Ab(nM))
25G7-Fab
ADC-2
TF-1 cells were co-incubated with IL-13
Light absorption value
log(Ab(nM))
25G7-Fab
ADC-2

*FIG. 2*

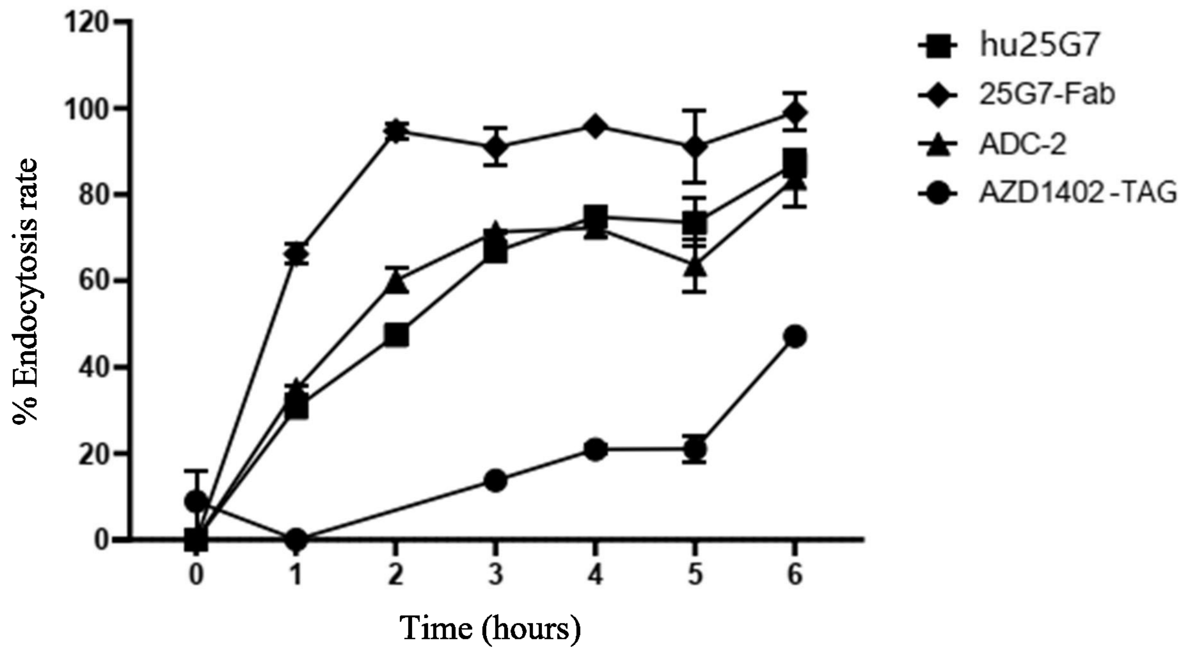
% Endocytosis rate
Time (hours)
hu25G7
25G7-Fab
ADC-2
AZD1402-TAG

*FIG. 3*

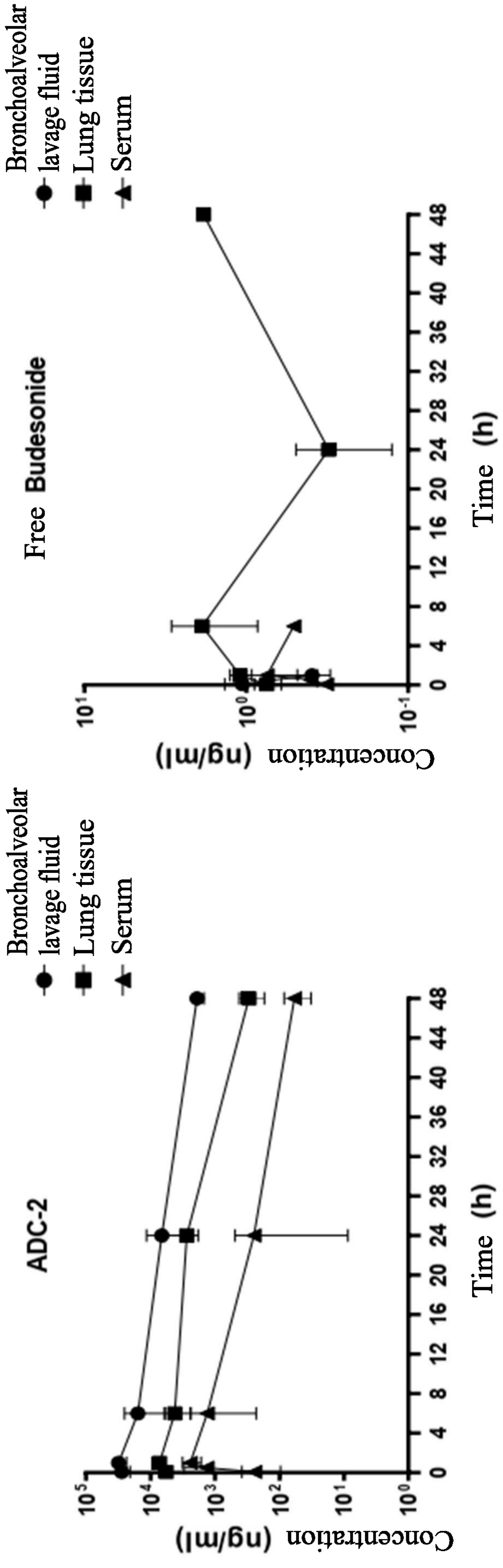
ADC-2
Concentration (ng/ml)
$10^5$
$10^4$
$10^3$
$10^2$
$10^1$
$10^0$
Time (h)
0 4 8 12 16 20 24 28 32 36 40 44 48
Bronchoalveolar lavage fluid
Lung tissue
Serum
Free Budesonide
Concentration (ng/ml)
$10^1$
$10^0$
$10^{-1}$
Time (h)
0 4 8 12 16 20 24 28 32 36 40 44 48
Bronchoalveolar lavage fluid
Lung tissue
Serum

*FIG. 4*

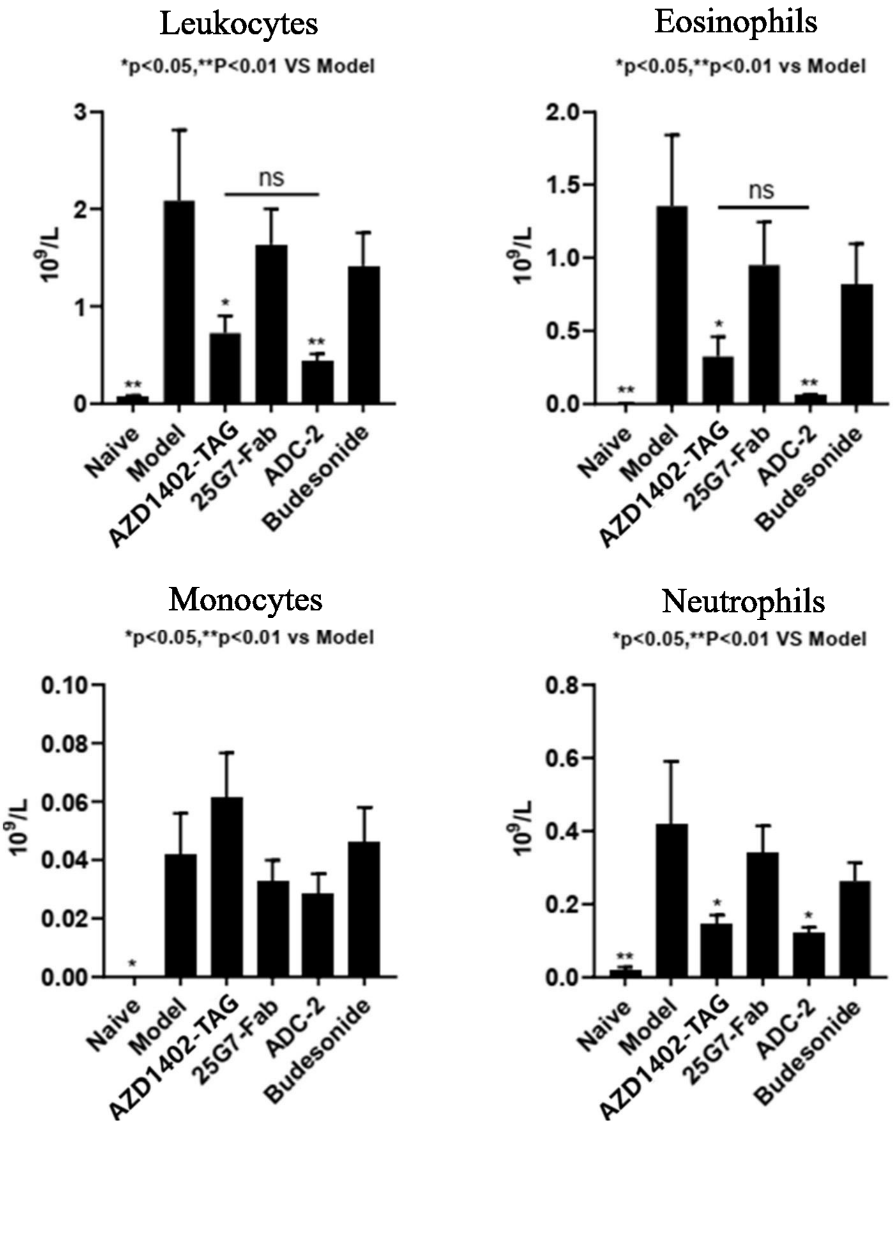
Leukocytes
*p<0.05,**P<0.01 VS Model
10^9/L
ns
Eosinophils
*p<0.05,**p<0.01 vs Model
Monocytes
*p<0.05,**p<0.01 vs Model
Neutrophils
*p<0.05,**P<0.01 VS Model
Naive
Model
AZD1402-TAG
25G7-Fab
ADC-2
Budesonide

*FIG. 5*

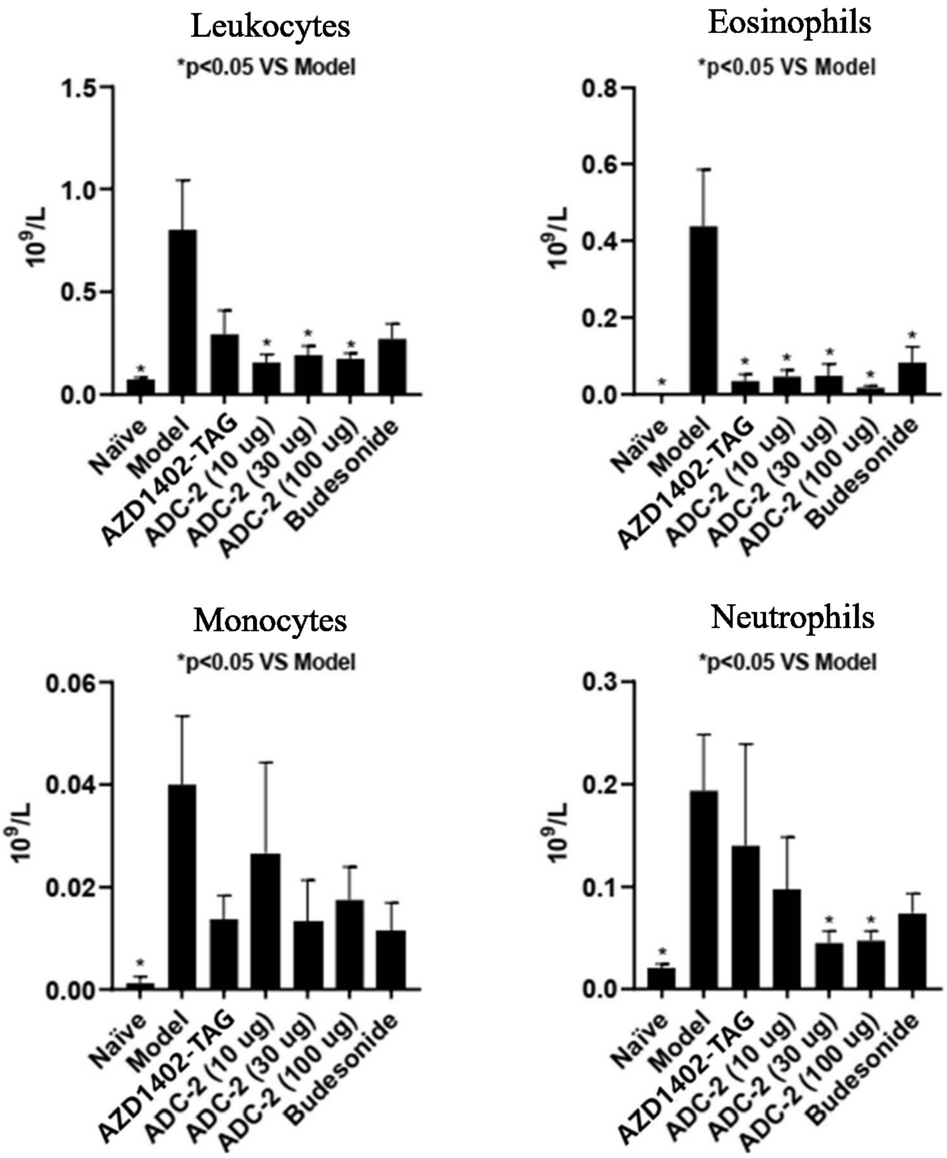
Leukocytes
*p<0.05 VS Model
1.5
1.0
0.5
0.0
10^9/L
Eosinophils
*p<0.05 VS Model
0.8
0.6
0.4
0.2
0.0
Monocytes
*p<0.05 VS Model
0.06
0.04
0.02
0.00
Neutrophils
*p<0.05 VS Model
0.3
0.2
0.1
0.0
Naïve
Model
AZD1402-TAG
ADC-2 (10 ug)
ADC-2 (30 ug)
ADC-2 (100 ug)
Budesonide

*FIG. 6*

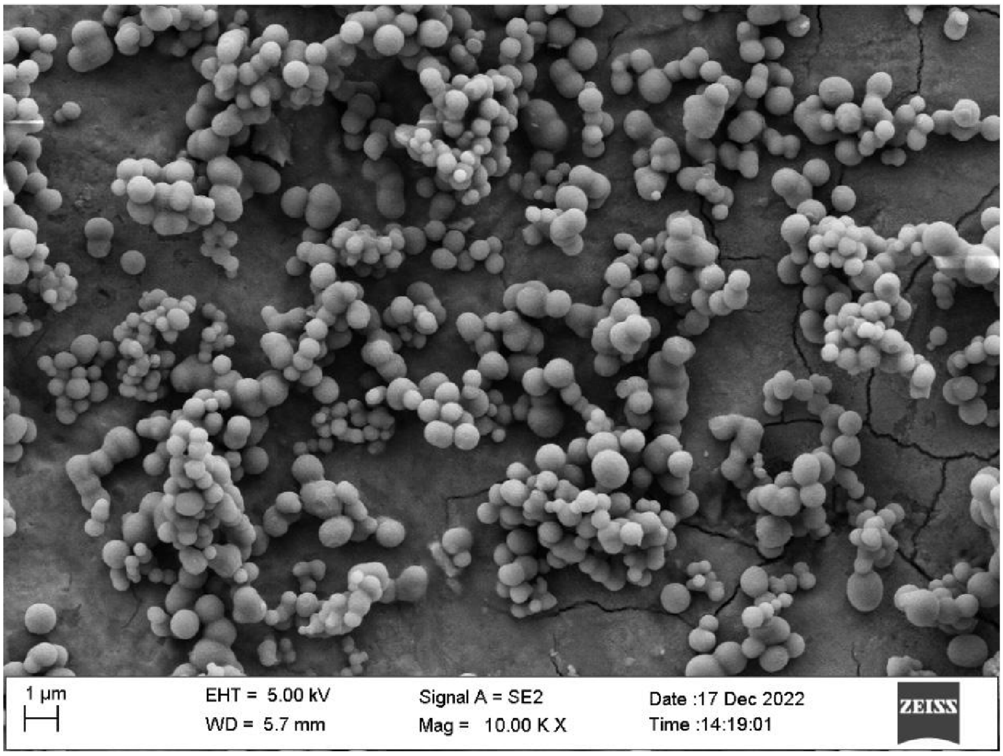
1 µm
EHT = 5.00 kV
WD = 5.7 mm
Signal A = SE2
Mag = 10.00 K X
Date :17 Dec 2022
Time :14:19:01
ZEISS

*FIG. 7*

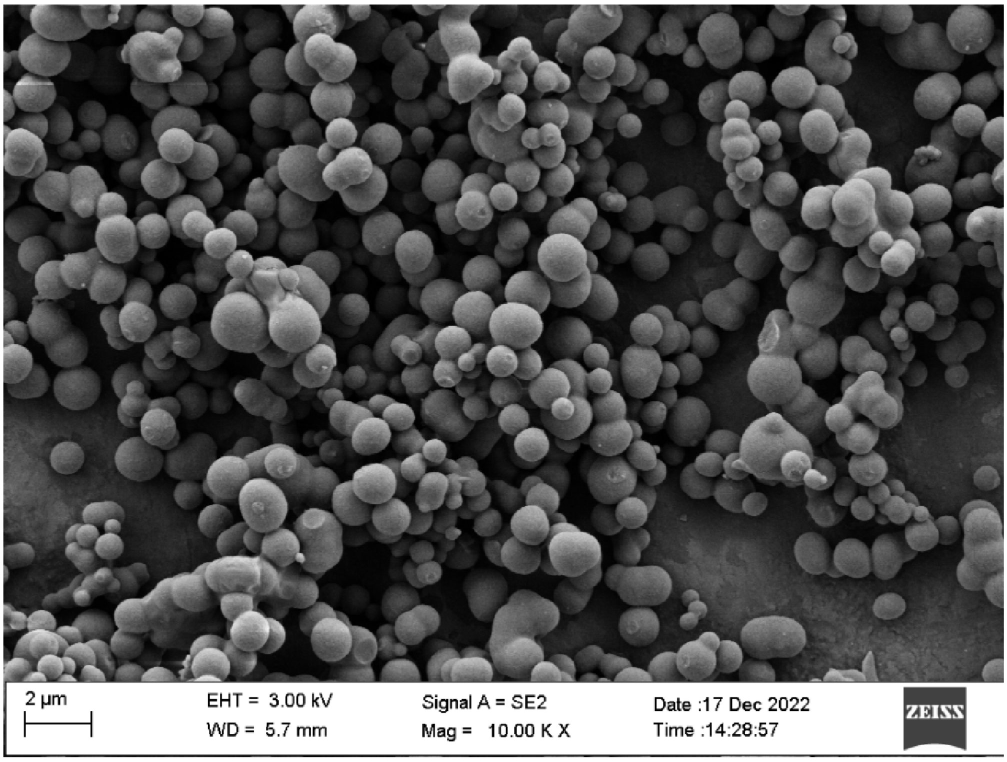
2 µm
EHT = 3.00 kV
WD = 5.7 mm
Signal A = SE2
Mag = 10.00 K X
Date :17 Dec 2022
Time :14:28:57
ZEISS

*FIG. 8*

**INTERNATIONAL SEARCH REPORT**

International application No.
**PCT/CN2024/107802**

**A. CLASSIFICATION OF SUBJECT MATTER**

A61K 47/68(2017.01)i；A61K31/58(2006.01)i；C07K16/22(2006.01)i；A61P37/00(2006.01)i

According to International Patent Classification (IPC) or to both national classification and IPC

**B. FIELDS SEARCHED**

Minimum documentation searched (classification system followed by classification symbols)

IPC：A61K，C07K，A61P

Documentation searched other than minimum documentation to the extent that such documents are included in the fields searched

Electronic data base consulted during the international search (name of data base and, where practicable, search terms used)

WPABSC; CNTXT; CNABS; WPABS; DWPI; ENTXTC: VCN; Web of Science; CNKI; 万方, WANFANG: STNext: 抗IL-4R, 白介素, 抗体, 偶联, 缀合, 布地奈德, 糖皮质激素, 聚乙二醇, 甘氨酰甘氨酰-L-苯丙氨酸叔丁酯, 生物序列检索, 结构检索, 哮喘, 过敏性皮肤病, ADC, Antibody, Drug, Conjugate, Asthma, Allergic dermatose?, Budesonide, Glucocorticoid

**C. DOCUMENTS CONSIDERED TO BE RELEVANT**

| Category* | Citation of document, with indication, where appropriate, of the relevant passages | Relevant to claim No. |
|---|---|---|
| PX | WO 2023143351 A1 (SHANGHAI SHENGDI PHARMACEUTICAL CO., LTD. et al.) 03 August 2023 (2023-08-03)<br>claims 1-21 | 1-21 |
| PY | CN 118236514 A (TIANJIN PHARMACEUTICAL BIOTECHNOLOGY CO., LTD.) 25 June 2024 (2024-06-25)<br>claims 1-10 | 1-21 |
| Y | CN 113905767 A (REGENERON PHARMACEUTICALS INC.) 07 January 2022 (2022-01-07)<br>claim 72, and description, page 16, paragraphs [0175], [0185], and [0321] | 1-21 |
| Y | WO 2020038454 A1 (JIANGSU HENGRUI MEDICINE CO., LTD. et al.) 27 February 2020 (2020-02-27)<br>claims 6, 8, 16, and 17, and description, SEQ ID NOs: 37, 43, 44, and 45 | 1-21 |
| Y | CN 110464847 A (DAIICHI SANKYO CO., LTD.) 19 November 2019 (2019-11-19)<br>description, embodiment 9 | 1-21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 October 2024** | **29 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/107802

C. DOCUMENTS CONSIDERED TO BE RELEVANT

| Category* | Citation of document, with indication, where appropriate, of the relevant passages | Relevant to claim No. |
|---|---|---|
| A | CN 111417410 A (ABBVIE INC.) 14 July 2020 (2020-07-14) claims 1-11 | 1-21 |
| A | CN 105307676 A (F. HOFFMANN-LA ROCHE AG) 03 February 2016 (2016-02-03) claims 1-68, and description, paragraph 0320 | 1-21 |

Form PCT/ISA/210 (second sheet) (July 2022)

**INTERNATIONAL SEARCH REPORT**

International application No.
**PCT/CN2024/107802**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/107802**

| Patent document cited in search report | Publication date (day/month/year) | Patent family member(s) | Publication date (day/month/year) |
|---|---|---|---|
| WO 2023143351 A1 | 03 August 2023 | None | |
| CN 118236514 A | 25 June 2024 | None | |
| CN 113905767 A | 07 January 2022 | BR 112021013464 A2 | 21 September 2021 |
| | | SG 11202107212 QA | 29 July 2021 |
| | | CA 3125998 A1 | 16 July 2020 |
| | | KR 20210114008 A | 17 September 2021 |
| | | EP 3908323 A2 | 17 November 2021 |
| | | IL 284673 A | 31 August 2021 |
| | | JP 2022517767 A | 10 March 2022 |
| | | WO 2020146541 A2 | 16 July 2020 |
| | | WO 2020146541 A3 | 13 August 2020 |
| | | AU 2020205662 A1 | 22 July 2021 |
| | | MA 53772 A1 | 31 October 2022 |
| | | MA 53772 B1 | 31 August 2023 |
| | | MA 59295 A1 | 31 July 2023 |
| | | MX 2021008114 A | 05 August 2021 |
| | | CL 2021001790 A1 | 01 July 2022 |
| | | CO 2021010315 A2 | 19 August 2021 |
| WO 2020038454 A1 | 27 February 2020 | None | |
| CN 110464847 A | 19 November 2019 | BR 112016013482 A2 | 03 October 2017 |
| | | BR 112016013482 B1 | 19 April 2022 |
| | | IL 278891 A | 31 January 2021 |
| | | IL 278891 B1 | 01 February 2024 |
| | | IL 278891 B2 | 01 June 2024 |
| | | JP 2022180504 A | 06 December 2022 |
| | | JP 7467557 B2 | 15 April 2024 |
| | | KR 20220025176 A | 03 March 2022 |
| | | KR 102417312 B1 | 05 July 2022 |
| | | KR 20210088745 A | 14 July 2021 |
| | | KR 102314913 B1 | 19 October 2021 |
| | | KR 20230162159 A | 28 November 2023 |
| | | PH 12016500904 A1 | 11 July 2016 |
| | | AU 2022203818 A1 | 23 June 2022 |
| | | AU 2020200596 A1 | 20 February 2020 |
| | | AU 2020200596 B2 | 03 March 2022 |
| | | TW 201834692 A | 01 October 2018 |
| | | TWI 661839 B | 11 June 2019 |
| | | HRP 20190431 T1 | 19 April 2019 |
| | | JP 2024081770 A | 18 June 2024 |
| | | RU 2016123597 A | 02 March 2018 |
| | | RU 2016123597 A3 | 12 October 2018 |
| | | RU 2683780 C2 | 02 April 2019 |
| | | EP 3466976 A1 | 10 April 2019 |
| | | EP 3466976 B1 | 08 September 2021 |
| | | SG 10201800210 TA | 27 February 2018 |
| | | LTPA 2021515 I1 | 10 August 2021 |
| | | LTC 3101032 I2 | 25 October 2022 |
| | | IL 310627 A | 01 April 2024 |
| | | NL 301117 I1 | 19 July 2021 |
| | | NL 301117 I2 | 29 July 2021 |

Form PCT/ISA/210 (patent family annex) (July 2022)

**INTERNATIONAL SEARCH REPORT**
**Information on patent family members**

International application No.
**PCT/CN2024/107802**

| Patent document cited in search report | Publication date (day/month/year) | Patent family member(s) | | | Publication date (day/month/year) |
|---|---|---|---|---|---|
| | | JP | 2019112403 | A | 11 July 2019 |
| | | JP | 6665325 | B2 | 13 March 2020 |
| | | ES | 2895254 | T3 | 18 February 2022 |
| | | CY | 1121573 | T1 | 29 May 2020 |
| | | US | 2024026030 | A1 | 25 January 2024 |
| | | EP | 3101032 | A1 | 07 December 2016 |
| | | EP | 3101032 | A4 | 06 September 2017 |
| | | EP | 3101032 | B1 | 16 January 2019 |
| | | LT | 3101032 | T | 25 March 2019 |
| | | TW | 202237113 | A | 01 October 2022 |
| | | TWI | 796243 | B | 11 March 2023 |
| | | US | 2020385486 | A1 | 10 December 2020 |
| | | US | 11584800 | B2 | 21 February 2023 |
| | | TW | 202322817 | A | 16 June 2023 |
| | | TW | 202237191 | A | 01 October 2022 |
| | | TWI | 796245 | B | 11 March 2023 |
| | | KR | 20210127803 | A | 22 October 2021 |
| | | KR | 102362920 | B1 | 14 February 2022 |
| | | HUE | 057464 | T2 | 28 May 2022 |
| | | MX | 2016006498 | A | 04 August 2016 |
| | | JP | 2021169493 | A | 28 October 2021 |
| | | JP | 7146031 | B2 | 03 October 2022 |
| | | EP | 4212552 | A1 | 19 July 2023 |
| | | JP | 2020097617 | A | 25 June 2020 |
| | | JP | 6914380 | B2 | 04 August 2021 |
| | | RS | 58415 | B1 | 30 April 2019 |
| | | HUE | 044389 | T2 | 28 October 2019 |
| | | FR | 2C1030 | I1 | 27 August 2021 |
| | | FR | 2C1030 | I2 | 08 April 2022 |
| | | SI | 3101032 | T1 | 28 February 2019 |
| | | EP | 3973995 | A1 | 30 March 2022 |
| | | RU | 2019107788 | A | 17 May 2019 |
| | | LT | 3466976 | T | 10 November 2021 |
| | | RS | 62618 | B1 | 31 December 2021 |
| | | TW | 202120088 | A | 01 June 2021 |
| | | TWI | 789700 | B | 11 January 2023 |
| | | WO | 2015115091 | A1 | 06 August 2015 |
| | | TW | 201934145 | A | 01 September 2019 |
| | | TWI | 722422 | B | 21 March 2021 |
| | | PT | 3466976 | T | 27 October 2021 |
| | | ES | 2727351 | T3 | 15 October 2019 |
| | | KR | 20210040181 | A | 12 April 2021 |
| | | KR | 102275925 | B1 | 12 July 2021 |
| | | MX | 2020010682 | A | 06 November 2020 |
| | | US | 2016333112 | A1 | 17 November 2016 |
| | | US | 10155821 | B2 | 18 December 2018 |
| | | KR | 20220154251 | A | 21 November 2022 |
| | | KR | 102510128 | B1 | 14 March 2023 |
| | | JP | 6105183 | B1 | 29 March 2017 |
| | | JP | 2017105789 | A | 15 June 2017 |

Form PCT/ISA/210 (patent family annex) (July 2022)

**INTERNATIONAL SEARCH REPORT**
**Information on patent family members**

International application No.
**PCT/CN2024/107802**

| Patent document cited in search report | Publication date (day/month/year) | Patent family member(s) | Publication date (day/month/year) |
|---|---|---|---|
| | | SI 3466976 T1 | 31 December 2021 |
| | | PT 3101032 T | 03 April 2019 |
| | | NO 2021027 I1 | 06 July 2021 |
| | | JP 2017095457 A | 01 June 2017 |
| | | JP 6466895 B2 | 06 February 2019 |
| | | KR 20180122038 A | 09 November 2018 |
| | | KR 102190548 B1 | 14 December 2020 |
| | | HRP 20211848 T1 | 04 March 2022 |
| | | ZA 201602802 B | 27 September 2017 |
| | | HUS 2100025 I1 | 28 July 2021 |
| | | KR 20230113822 A | 01 August 2023 |
| | | KR 102606930 B1 | 29 November 2023 |
| | | TW 201613649 A | 16 April 2016 |
| | | TWI 627967 B | 01 July 2018 |
| | | IL 245252 A0 | 30 June 2016 |
| | | IL 245252 B | 30 June 2019 |
| | | KR 20200140932 A | 16 December 2020 |
| | | KR 102238848 B1 | 09 April 2021 |
| | | KR 20220100994 A | 18 July 2022 |
| | | KR 102465042 B1 | 09 November 2022 |
| | | CY 1124774 T1 | 25 November 2022 |
| | | KR 20230042126 A | 27 March 2023 |
| | | KR 102557062 B1 | 18 July 2023 |
| | | NZ 719202 A | 25 February 2022 |
| | | CY 2021018 I2 | 15 October 2021 |
| | | IL 266790 A | 31 July 2019 |
| | | JP 6046301 B1 | 14 December 2016 |
| | | JP 2017036285 A | 16 February 2017 |
| | | PL 3101032 T3 | 31 July 2019 |
| | | DK 3101032 T3 | 29 April 2019 |
| | | JP 5998289 B2 | 28 September 2016 |
| | | JPWO 2015115091 A1 | 23 March 2017 |
| | | TW 202237114 A | 01 October 2022 |
| | | TWI 796244 B | 11 March 2023 |
| | | CA 2928794 A1 | 06 August 2015 |
| | | CA 2928794 C | 13 August 2019 |
| | | TW 202237115 A | 01 October 2022 |
| | | TWI 796246 B | 11 March 2023 |
| | | US 2019077880 A1 | 14 March 2019 |
| | | US 11795236 B2 | 24 October 2023 |
| | | KR 20160113099 A | 28 September 2016 |
| | | KR 101916553 B1 | 07 November 2018 |
| | | SG 11201603960 XA | 28 July 2016 |
| | | PL 3466976 T3 | 20 December 2021 |
| | | AU 2015212265 B2 | 02 January 2020 |
| | | DK 3466976 T3 | 13 December 2021 |
| CN 111417410 A | 14 July 2020 | LT 3658192 T | 12 July 2021 |
| | | CA 3082356 A1 | 06 June 2019 |
| | | CL 2020001452 A1 | 11 September 2020 |
| | | ZA 202003325 B | 28 July 2021 |

Form PCT/ISA/210 (patent family annex) (July 2022)

**INTERNATIONAL SEARCH REPORT**
**Information on patent family members**

International application No.
**PCT/CN2024/107802**

| Patent document cited in search report | Publication date (day/month/year) | Patent family member(s) | Publication date (day/month/year) |
|---|---|---|---|
| | | PT 3658192 T | 25 June 2021 |
| | | UY 37991 A | 28 June 2019 |
| | | JP 2021054845 A | 08 April 2021 |
| | | RS 61994 B1 | 30 July 2021 |
| | | CL 2021001075 A1 | 15 October 2021 |
| | | SI 3658192 T1 | 31 August 2021 |
| | | MA 49796 A | 03 June 2020 |
| | | AU 2018374634 A1 | 28 May 2020 |
| | | JP 6813712 B2 | 13 January 2021 |
| | | JP 2021501124 A | 14 January 2021 |
| | | HRP 20210917 T1 | 03 September 2021 |
| | | KR 20200095477 A | 10 August 2020 |
| | | US 2021015938 A1 | 21 January 2021 |
| | | PE 20201286 A1 | 24 November 2020 |
| | | CY 1124230 T1 | 27 May 2022 |
| | | PL 3658192 T3 | 18 October 2021 |
| | | IL 274651 A | 30 June 2020 |
| | | IL 274651 B | 29 April 2021 |
| | | DK 3658192 T3 | 21 June 2021 |
| | | DOP 2020000116 A | 31 August 2020 |
| | | ECSP 20029001 A | 30 June 2020 |
| | | CO 2020006446 A2 | 09 June 2020 |
| | | EP 3658192 A1 | 03 June 2020 |
| | | EP 3658192 B1 | 28 April 2021 |
| | | ES 2877659 T3 | 17 November 2021 |
| | | MX 2020005583 A | 25 November 2020 |
| | | WO 2019106609 A1 | 06 June 2019 |
| | | UA 126595 C2 | 02 November 2022 |
| | | TW 201924723 A | 01 July 2019 |
| | | TWI 803542 B | 01 June 2023 |
| | | US 2019167804 A1 | 06 June 2019 |
| | | US 10772970 B2 | 15 September 2020 |
| | | CR 20200239 A | 21 September 2020 |
| | | BR 112020010694 A2 | 10 November 2020 |
| | | EP 3884962 A1 | 29 September 2021 |
| | | HUE 054428 T2 | 28 September 2021 |
| | | RU 2020117698 A3 | 04 January 2022 |
| | | SG 11202004867 WA | 29 June 2020 |
| CN 105307676 A | 03 February 2016 | CA 2905223 A1 | 09 October 2014 |
| | | JP 2016522168 A | 28 July 2016 |
| | | BR 112015024553 A2 | 24 October 2017 |
| | | AR 095774 A1 | 11 November 2015 |
| | | TW 201518321 A | 16 May 2015 |
| | | WO 2014165771 A2 | 09 October 2014 |
| | | WO 2014165771 A3 | 27 November 2014 |
| | | US 2016207995 A1 | 21 July 2016 |
| | | RU 2015141529 A | 15 May 2017 |
| | | MX 2015013901 A | 11 December 2015 |
| | | HK 1220919 A1 | 19 May 2017 |
| | | EP 2981286 A2 | 10 February 2016 |

Form PCT/ISA/210 (patent family annex) (July 2022)

**INTERNATIONAL SEARCH REPORT**
**Information on patent family members**

International application No.
**PCT/CN2024/107802**

| Patent document cited in search report | Publication date (day/month/year) | Patent family member(s) | Publication date (day/month/year) |
|---|---|---|---|
| | | EP 2981286 A4 | 24 August 2016 |
| | | KR 20150139905 A | 14 December 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023109382848
- WO 2010053751 A
- WO 2001092340 A
- WO 2008054606 A
- WO 2014031610 A
- WO 2020038454 A
- WO 2017210471 A
- WO 2019106609 A
- WO 2019136487 A
- WO 2020038454 A1
- CN 2023073057 W
- WO 2020200960 A1


### Non-patent literature cited in the description


- Pharmacopoeia of the People's Republic of China. 2015
- *J. biol. chem*, 1968, vol. 243, 3558
- **KABAT, E.A. et al.** Sequences of Proteins of Immunological Interest. 1991
- **QUEEN et al.** *Proc. Natl. Acad. Sci. USA*, 1991, vol. 88, 2869
- **JONES et al.** *Nature*, 1986, vol. 321, 522
- **RIECHMANN et al.** *Nature*, 1988, vol. 332, 323-327
- **VERHOEYEN et al.** *Science*, 1988, vol. 239, 1534
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur.J.Immuno*, 2001, vol. 1 (31), 94-106
- **HU et al.** *Cancer Res.*, 1996, vol. 56, 3055-3061
- **KIPRIYANOV et al.** *J.Mol.Biol.*, 1999, vol. 293, 41-56
- **ROOVERS et al.** *Cancer Immunol*, 2001
- Sequences of proteins of immunological interest. **KABAT E.A et al.** NIH Publication 91-3242. 1991
- **KABAT et al.** Sequences of Proteins of Immunological Interest. National Institutes of Health, 1991
- **AL-LAZIKANI et al.** *JMB*, 1997, vol. 273, 927-948
- **LEFRANC M. P**. *Immunologist*, 1999, vol. 7, 132-136
- **LEFRANC, M.P. et al.** *Dev. Comp. Immunol*, 2003, vol. 27, 55-77
- Epitope Mapping Protocols in Methods in Molecular Biology. 1996, vol. 66
- *the Journal of Immunoglobulins*, 2001, ISBN 012441351
- Antibody Technology Laboratory Manual or Molecular Cloning: A Laboratory Manual. Cold Spring Harbor Laboratory
- *Tetrahedron*, 2018, vol. 74 (15), 1951-1956
- Determination of Aerodynamic Properties of Fine Particles in Inhalation Preparations. the Chinese Pharmacopoeia. 2015, vol. IV